# EUROPÄISCHE PATENTSCHRIFT

(11) **EP 1 671 673 B1**
(45) Veröffentlichungstag und Bekanntmachung des Hinweises auf die Patenterteilung: **22.04.2009**
(21) Anmeldenummer: 05026915.8
(22) Anmeldetag: 09.12.2005
(51) Int. Cl.: A61Q 1/02, A61K 8/06, A61K 8/58, A61K 8/89, A61K 8/92

(54) **Pigmenthaltige Wasser-in-Siliconöl-Emulsion zur Verbesserung des Erscheinungsbildes der Haut**
Pigments containing water-in-siliconoil-emulsion for improving the appearance of the skin
Composition contenant des pigments sous forme d'émulsion eau-dans-huile de silicone pour ameliorer l'apparence de la peau

(30) Priorität: 20.12.2004 DE 102004062430
(43) Veröffentlichungstag der Anmeldung: 21.06.2006
(73) Patentinhaber: Henkel AG & Co. KGaA, 40589 Düsseldorf (DE)
(72) Erfinder: Döring, Thomas, 41542 Dormagen (DE); Waldmann-Laue, Marianne, 40789 Monheim (DE); Träger, Anemone, 40599 Düsseldorf (DE)

(56) Entgegenhaltungen:
- EP-A- 0 846 461
- US-A- 5 800 816
- US-A1- 2004 175 345

## Beschreibung

Die Erfindung betrifft topische kosmetische Zusammensetzungen auf der Basis einer pigmenthaltigen Wasser-in-Siliconöl-Emulsion, deren Ölphase mit einer hochschmelzenden Lipidkomponente verdickt ist, die das Erscheinungsbild der Haut verbessern, indem sie Hautunebenheiten, z. B. feine Linien, Fältchen und Falten, Narben, Pickel oder grobe Hautporen, optisch kaschieren.

Topische kosmetische Zusammensetzungen auf der Basis von Wasser-in-Siliconöl-Emulsionen, die eine bei Raumtemperatur feste Lipidkomponente enthalten, sind im Stand der Technik bekannt.
US 5,523,091 offenbart pigmenthaltige Wasser-in-Siliconöl-Emulsionen, enthaltend einen Siliconemulgator und eine Wachskomponente mit der INCI-Bezeichnung Acetylated Glycol Stearate and Tristearin, erhältlich unter dem Handelsnamen Unitwix von Guardian Laboratories. Der Rohstoff Unitwix weist laut Herstellerangaben einen Schmelzpunkt von 70 - 75°C auf. Dieser Publikation ist für den Fachmann kein Hinweis darauf zu entnehmen, dass sich derartige Zusammensetzungen mit speziell ausgewählten Pigmenten, insbesondere solchen mit einer mittleren Partikelgröße von 0,5 - 25 µm, die im Wellenlängenbereich von 400 - 800 nm diffuse Lichtstreuungseigenschaften und einen Transmissionswert von 60 bis mindestens 95 % aufweisen, zu einer verbesserten optischen Faltenkaschierung eignen.
EP 374 332 B1 beansprucht feste Wasser-in-Siliconöl-Emulsionen mit einem Wachsgehalt von 3 - 30 Gew.-%, die einen Siliconemulgator und Pigmente enthalten. Aber auch dieser Publikation entnimmt der Fachmann keinen Hinweis darauf, dass sich derartige Zusammensetzungen im Zusammenwirken mit den oben genannten ausgewählten Pigmenten, zu einer verbesserten optischen Faltenkaschierung eignen könnten.
WO 03/022235 A1 offenbart Wasser-in-Siliconöl-Emulsionen mit Siliconwachsen, z. B. Cetyldimethicone und Stearyldimethicone. Diese Siliconwachse sind entweder bei Raumtemperatur flüssig oder aber weisen Schmelzpunkte deutlich unterhalb von 60 °C auf.
GB 2 065 587 offenbart eine Wasser-in-Siliconöl-Emulsion mit 18 % Siliconöl, Siliconemulgator und 5 % Lanolin. Auch hier findet der Fachmann keinen Hinweis auf eine optische Faltenkaschierung. Lanolin weist üblicherweise einen Schmelzpunkt von 38 - 44 °C auf.
Die nicht-therapeutische Behandlung von Hautunebenheiten durch kosmetische Zusammensetzungen ist im Stand der Technik bereits bekannt. So dienen partikelförmige Materialien, wie Talkum, Stärke und Stärkederivate, Cellulosepulver, Polymerpulver (z. B. Nylonpulver, erhältlich unter der Handelsbezeichnung Orgasol) oder farbige Pigmente dazu, Hautunebenheiten durch farbliches Angleichen zwischen unebenen Stellen und umgebender Haut zu verdecken.
Um eine länger anhaltende abdeckende Wirkung zu erreichen, muss der Pigmentgehalt in den bekannten Zusammensetzungen einen Anteil von bis zu 20 Gew.% an der kosmetischen Zusammensetzung ausmachen. Derartige Zusammensetzungen aus dem Stand der Technik weisen gewisse Nachteile auf. So muss der relativ hohe Pigmentgehalt durch einen hohen Gehalt an Emulgatoren lagerstabil gemacht werden, damit die Pigmente auch bei längerer Lagerung gleichmäßig dispergiert bleiben und sich nicht unter dem Einfluss der Schwerkraft absetzen. Ein hoher Gehalt an Emulgator kann aber, je nach Art des Emulgators, zu Unverträglichkeitsreaktionen der Haut führen bzw. die Anwendungsmöglichkeit im besonders empfindlichen Augenbereich einschränken. Weiterhin schränkt ein hoher Pigmentgehalt den Tragekomfort einer Zusammensetzung beträchtlich ein und beeinträchtigt den Feuchtigkeitsgehalt der Haut, außerdem erfolgt häufig eine Migration der Pigmentpartikel in die Vertiefungen des Hautreliefs, z. B. in die Mimikfältchen, hinein und macht diese sichtbar. Ohne an diese Vermutung gebunden sein zu wollen, wird vermutet, dass die unerwünschte Migration der Pigmente auf der Haut begünstigt wird in alleiniger Anwesenheit ölphasenverdickende Lipid- oder Wachskomponenten mit einem Schmelzpunkt im Bereich der Hauttemperatur.
Zur optischen Faltenbehandlung wurde in der Druckschrift US 4,255,416 vorgeschlagen, eine pigmenthaltige Zusammensetzung mit einem filmbildenden Polymer und einem höheren Anteil an flüchtigem Lösemittel auf die Haut aufzutragen. Der Film, der sich nach Verdunsten des Lösemittels auf der Haut bildet, soll zu einer mechanischen Straffung der Haut führen und dadurch feine Fältchen und Linien glätten. Derartige Zusammensetzungen weisen häufig nur einen geringen Tragekomfort auf.
In EP 1 136 064 A2 sind kosmetische Zusammensetzungen offenbart, die in einem geeignetem Träger ein vernetztes Silicon-Elastomer und sphärische Partikel mit einer Differenz zwischen maximaler und minimaler Partikelgröße von etwa 24 µm enthalten.

Als geeignete Partikel sind z. B. Kieselsäuren (Silica) offenbart. Weiterhin werden Verfahren zur Verbesserung des ästhetischen Erscheinungsbildes der Haut, insbesondere zur Verbesserung des Erscheinungsbilds der Hauttextur, zur Verminderung des Erscheinungsbildes von feinen Linien und Falten, zur Verbesserung der Hautfarbe und zur Verfeinerung des Erscheinungsbildes der Porengröße offenbart. Das Silicon-Elastomer soll dabei Hautfältchen ausfüllen und ebnen; die sich darauf ablagernden Mikropartikel sollen durch Lichtreflexionen das Erscheinungsbild der Haut verbessern. Konkrete Angaben zum Emulsionstyp, in den die Pigmente eingearbeitet sind, sind für den Fachmann nicht zu entnehmen.
US 5,919,468 offenbart eine Wasser-in-Siliconöl-Emulsion, enthaltend ein Siliconöl, einen Siliconemulgator und ein Siliconelastomer. Die Zusammensetzung kann zum optischen Ausgleich des Hautreliefs verwendet werden. Allerdings wurde festgestellt, dass die optische Wirkung dieser Zusammensetzung noch verbesserungswürdig ist. Ohne an diese Theorie gebunden sein zu wollen, wird vermutet, dass der geringe Anteil des Siliconöls an der Fettphase, gegebenenfalls im Zusammenwirken mit dem Elastomergehalt, die Spreiteigenschaften und das Langzeittrageverhalten der Zusammensetzung ungünstig beeinflusst, so dass die Pigmente mit der Zeit in die Vertiefungen des Hautreliefs migrieren und diese sichtbar machen.
US 5800816 offenbart eine transferresistente Make-up-Zubereitung in Form einer Wasser- und Öl-Emulsion, umfassend 0,1 bis 60 Gew.-% trimethyliertes Silica, 0,1 bis 60 Gew.-% eines flüchtigen Lösungsmittels mit einer Viskosität von 0,5 bis 20 cPs bei 25 °C; ausgewählt aus Cyclomethiconen mit 1 - 7 Dimethylsiloxan-Einheiten, linearen Siliconen der Formel (CH₃)₃SiO-(-Si(CH₃)₂-O-)ₙ-Si(CH₃)₃ mit n = 0 - 7, geraden oder verzweigten Kohlenwasserstoffen mit 8-20 Kohlenstoffatomen und Ethanol, 0,1 bis 60 Gew.-% eines nicht-flüchtigen Öls, ausgewählt aus Dimethicone, Dimethicone Copolyolen und Mischungen hiervon, 0,1 bis 80 Gew.-% partikulären Pigmenten oder Pulvern sowie 0,1-50 Gew.-% Wasser.
EP 846 461 A1 offenbart Öl-in-Wasser-Emulsionen, enthaltend einen alpha-Monoalkylglycerylether, ein Wachs, ein Siliconöl, wobei das Siliconöl mindestens 10 Gew.-% der Ölphase (ohne alpha-Monoalkylglycerylether und Wachse) ausmacht und wobei diese Emulsionen Pigmente enthalten können.

Aufgabe der vorliegenden Erfindung war es, topische kosmetische Zusammensetzungen zur Verbesserung des Hauterscheinungsbildes durch optisches Kaschieren von Hautunebenheiten bereitzustellen, mit denen sich ein gegenüber dem Stand der Technik verbessertes Hauterscheinungsbild erzielen lässt.
Eine weitere Aufgabe der vorliegenden Erfindung war es, topische kosmetische Zusammensetzungen zur Verbesserung des Hauterscheinungsbildes durch optisches Kaschieren von Hautunebenheiten bereitzustellen, die gegenüber dem Stand der Technik verbesserte Anwendungseigenschaften aufweisen.
Eine weitere Aufgabe der vorliegenden Erfindung war es, topische kosmetische Zusammensetzungen zur Verbesserung des Hauterscheinungsbildes durch optisches Kaschieren von Hautunebenheiten bereitzustellen, die einen gegenüber dem Stand der Technik verbesserten Tragekomfort und/oder ein verbessertes Hautgefühl aufweisen.
Eine weitere Aufgabe der vorliegenden Erfindung war es, topische kosmetische Zusammensetzungen zur Verbesserung des Hauterscheinungsbildes durch optisches Kaschieren von Hautunebenheiten bereitzustellen, die gegenüber dem Stand der Technik verbesserte, das heißt verringerte, Migrationseigenschaften aufweisen.

Überraschend wurde nun gefunden, dass Wasser-in-Siliconöl-Emulsionen mit einem hohen Siliconölanteil, deren Ölphase mit einer hochschmelzenden Lipidkomponente verdickt ist und die mit einem W/O-Siliconemulgator stabilisiert sind, zusammen mit spezifisch ausgewählten Pigmenten, die im Wellenlängenbereich von 400 - 800 nm diffuse Lichtstreuungseigenschaften und eine hohe Transmission aufweisen, die gestellten Aufgaben in hervorragender Weise lösen.

Gegenstand der vorliegenden Erfindung sind kosmetische Zusammensetzungen in Form einer Creme oder Lotion auf Basis einer Wasser-in-Siliconöl-Emulsion, die 15 - 35 Gew.-% einer Fettphase mit einem Anteil an mindestens einem Siliconöl von 70 - 95 Gew.%, bezogen auf das Gewicht der Fettphase, Cetyl PEG/PPG-10/1 Dimethicone als Wasser-in-Öl-Siliconemulgator, 50 - 90 Gew.-% Wasserphase, bezogen auf die Gesamtzusammensetzung, 0,5 bis weniger als 3 Gew.-% mindestens einer Lipid- oder Wachskomponente, wobei der Schmelzpunkt der Lipid- oder Wachskomponente oberhalb bei 60°C oder darüber liegt oder, falls mehrere Lipid- oder Wachskomponenten enthalten sind, der Schmelzpunkt dieser Mischung von Lipid- oder Wachskomponenten bei 60°C oder darüber liegt, und wobei die Lipid- oder Wachskomponente mit einem Schmelzpunkt von 60°C oder darüber ausgewählt ist aus Estern aus einem gesättigten, einwertigen C₁₆-C₆₀-Alkanol und einer gesättigten C₈-C₃₆-Monocarbonsäure, Glycerintriestern von gesättigten linearen C₁₈ - C₄₀-Carbonsäuren, die hydroxyliert sein können, Fruchtwachsen, Pflanzenwachsen, insbesondere Candelillawachs und Carnaubawachs, Insektenwachsen, insbesondere Bienenwachs, gesättigten linearen C₁₆ -, C₁₈ -, C₂₀ - und C₂₂ - C₄₀-Carbonsäuren, gehärtetem Ricinusöl, Ozokerit, Mikrowachsen, Ceresin, Paraffinwachsen, sowie solchen Mischungen der vorgenannten Substanzen, deren Schmelzpunkt bei 60°C oder darüber liegt, und 0,5 - 7 Gew.-% mindestens eines Pigments einer mittleren Partikelgröße von 0,5 - 25 µm, das im Wellenlängenbereich von 400 - 800 nm diffuse Lichtstreuungseigenschaften und einen Transmissionswert von 60 bis mindestens 95 % aufweist und das ausgewählt ist aus sphärischen Kieselsäurepartikeln, die mit Titandioxid (CI 77891) und Eisenoxid (CI. 77491) beschichtet sind, sphärischen Partikeln aus vernetztem Polymethylmethacrylat, sphärischen TiO₂-haltigen Kieselsäurepartikeln sowie Mischungen der genannten Pigmente, enthalten.

Die erfindungsgemäßen Zusammensetzungen enthalten eine Fettphase, die einen Anteil von 15 - 35 Gew.-%, besonders bevorzugt 20 - 32 Gew.-% und außerordentlich bevorzugt 25 - 32 Gew.-% an der Gesamtzusammensetzung ausmacht. Die erfindungsgemäß verwendeten W/O-Siliconemulgatoren und gegebenenfalls zusätzlich enthaltene W/O- und/oder O/W-Emulgatoren werden definitionsgemäß nicht zur Fettphase gerechnet. Zur Fettphase werden bei Raumtemperatur flüssige Ölkomponenten und bei Raumtemperatur feste Wachse und Fettkörper gezählt, weiterhin öllösliche Wirkstoffe, beispielsweise öllösliche organische UV-Filter, oder Parfümöle.
Die Fettphase weist einen Anteil von 70 - 95 Gew.%, jeweils bezogen auf das Gewicht der Fettphase, an mindestens einem Siliconöl auf. Bezogen auf die Gesamtzusammensetzung (Emulsion) beträgt der Anteil an Siliconöl/en demnach 15 - 32 Gew.-% und bevorzugt 20 - 32 Gew.-%. Geeignete Siliconöle sind bevorzugt ausgewählt aus flüchtigen und nichtflüchtigen Siliconölen, die cyclisch sein können, wie z. B. Octamethylcyclotetrasiloxan, Decamethylcyclopentasiloxan und Dodecamethylcyclohexasiloxan sowie Mischungen hiervon, wie sie z. B. in den Handelsprodukten DC 244, 245, 344 und 345 von Dow Corning enthalten sind, oder linear, z. B. Hexamethyldisiloxan (L₂), Octamethyltrisiloxan (L₃), Decamethyltetrasiloxan (L₄), beliebige Zweier- und Dreiermischungen aus L₂, L₃ und/oder L₄, wie sie z. B. in den Handelsprodukten DC 2-1184, Dow Corning® 200 (0, 65 cSt) und Dow Corning® 200 (1,5 cSt) von Dow Corning enthalten sind, sowie nichtflüchtige höhermolekulare lineare Dimethylpolysiloxane, im Handel erhältlich z. B. unter der Bezeichnung Dow Corning® 200 Fluid mit Viskositäten im Bereich von 5 - 100 cSt, bevorzugt 5 - 50 cSt oder auch 5 - 10 cSt, und Baysilon® 350 M oder die Produkte aus der Serie Belsil® DM von Wacker, beispielsweise ein Dimethicone mit einer Viskosität von 100 cSt, oder Phenyltrimethicone, beispielsweise der Rohstoff Dow Corning® 556 Fluid. Besonders bevorzugt sind Mischungen von flüchtigen und nichtflüchtigen Siliconölen, wobei man über das Mengenverhältnis dieser beiden Öltypen das Verdunstungs- bzw. Spreit-und Einziehverhalten und damit das gewünschte Hautgefühl einstellen kann. Außerordentlich bevorzugt ist ein Gewichtsverhältnis von flüchtigem/n zu nichtflüchtigem/n Siliconöl/en von 7 - 11 zu 1, noch bevorzugter 9 - 10 zu 1. Weitere erfindungsgemäß besonders bevorzugte Siliconöl-Kombinationen aus flüchtigen und nicht-flüchtigen Siliconölen enthalten Decamethylcyclopentasiloxan und Dimethicone mit einer Viskosität von mindestens 5 cSt, Dodecamethylcyclohexasiloxan und Dimethicone mit einer Viskosität von mindestens 5 cSt, Decamethylcyclopentasiloxan und Dodecamethylcyclohexasiloxan und Dimethicone mit einer Viskosität von mindestens 5 cSt, Hexamethyldisiloxan (L₂) und Dimethicone mit einer Viskosität von mindestens 5 cSt, Octamethyltrisiloxan (L₃) und Dimethicone mit einer Viskosität von mindestens 5 cSt, Decamethyltetrasiloxan und Dimethicone mit einer Viskosität von mindestens 5 cSt, das Handelsprodukt Dow Corning® DC 2-1184 und Dimethicone mit einer Viskosität von mindestens 5 cSt. Anstelle von Dimethicone mit einer Viskosität von mindestens 5 cSt kann als nicht-flüchtiges Siliconöl auch Phenyltrimethicone eingesetzt werden, beispielsweise der Rohstoff Dow Corning® 556 Fluid. Zusätzlich zu Dimethicone mit einer Viskosität von mindestens 5 cSt kann als nicht-flüchtiges Siliconöl auch Phenyltrimethicone eingesetzt werden.

Zur Fettphase der erfindungsgemäßen Zusammensetzungen zählt weiterhin mindestens eine Lipid- oder Wachskomponente mit einem Schmelzpunkt von 60°C oder darüber, bevorzugt 70°C oder darüber und besonders bevorzugt 78°C oder darüber, ausgewählt aus Estern aus einem gesättigten, einwertigen C₁₆-C₆₀-Alkanol und einer gesättigten C₈-C₃₆-Monocarbonsäure, Glycerintriestern von gesättigten linearen C₁₈ - C₄₀-Carbonsäuren, die hydroxyliert sein können, Fruchtwachsen, Pflanzenwachsen, insbesondere Candelillawachs und Carnaubawachs, Insektenwachsen, insbesondere Bienenwachs, künstlichen Bienenwachsen, z.B. dem Handelsprodukt Kesterwachs K 80 H von Koster Keunen, gesättigten linearen C₁₆ -, C₁₈ -, C₂₀ - und C₂₂ - C₄₀-Carbonsäuren, gehärtetem Ricinusöl, Ozokerit, Mikrowachsen, Ceresin, Paraffinwachsen, sowie solchen Mischungen der vorgenannten Substanzen, deren Schmelzpunkt bei 60°C oder darüber, bevorzugt 70°C oder darüber und besonders bevorzugt 78°C oder darüber liegt. Eine besonders bevorzugte Lipidkomponente ist gehärtetes Ricinusöl.
Die erfindungsgemäßen kosmetischen Zusammensetzungen enthalten die Lipid- oder Wachskomponente mit einem Schmelzpunkt von 60°C oder darüber in einer Gesamtmenge von 0,5 bis weniger als 3 Gew.-%, bevorzugt 1,0 **-** 2,5 Gew.-%, außerordentlich bevorzugt 2,0 Ges.-%, jeweils bezogen auf die Gesamtzusammensetzung.
Erfindungsgemäß besonders bevorzugte Zusammensetzungen sind dadurch gekennzeichnet, dass alle Bestandteile der Fettphase, die bei Raumtemperatur fest vorliegen, einen Schmelzpunkt von 60°C oder darüber, bevorzugt 70°C oder darüber und besonders bevorzugt 78°C oder darüber, aufweisen.
Bei der Auswahl der eingesetzten Menge an Lipid- oder Wachskomponente mit einem Schmelzpunkt von 60°C oder darüber ist zu beachten, dass die Zusammensetzung keine stiftartige Konsistenz erlangt, sondern eine viskose Creme oder Lotion bleibt. Hierbei sind Viskositäten im Bereich von 4000 - 3.000.000 mPas bei 20 - 25 °C bevorzugt. Die Messparameter für die Viskositätsbestimmung, insbesondere die Wahl der Spindel und der Umdrehungszahl, können dabei vom Fachmann anhand einschlägiger Handbücher der Viskosimeter-Hersteller, z. B. "More solutions to sticky problems" von Brookfield, für den jeweiligen Viskositätsbereich optimal bestimmt werden. Bevorzugt ist weiterhin, dass die Viskositätsbestimmungen unter Verwendung eines Helipath durchgeführt werden.

Die Fettphase kann in einer bevorzugten Ausführungsform der Erfindung weitere Ölkomponenten enthalten, ausgewählt aus:
- den Estern von linearen oder verzweigten gesättigten oder ungesättigten Fettalkoholen mit 2-30 Kohlenstoffatomen mit linearen oder verzweigten gesättigten oder ungesättigten Fettsäuren mit 2-30 Kohlenstoffatomen, die hydroxyliert sein können. Dazu zählen 2-Ethylhexylpalmitat (z. B. Cegesoft® C 24), Hexyldecylstearat (Eutanol® G 16), Hexyldecyllaurat, Isodecylneopentanoat, Isononylisononanoat, 2-Ethylhexylstearat, Isopropylmyristat, Isopropylpalmitat, Isopropylstearat, Isopropylisostearat, Isopropyloleat, Isooctylstearat, Isononylstearat, Isocetylstearat, Isononylisononanoat, Isotridecylisononanoat, Cetearylisononanot, 2-Ethylhexyllaurat, 2-Ethylhexylisostearat, 2-Ethylhexylcocoat, 2-Octyldodecylpalmitat, Butyloctansäure-2-butyloctanoat, Diisotridecylacetat, n-Butylstearat, n-Hexyllaurat, n-Decyloleat, Oleyloleat, Oleylerucat, Erucyloleat, Erucylerucat, Ethylenglycoldioleat und -dipalmitat;
- den Benzoesäureestern von linearen oder verzweigten C₈₋₂₂-Alkanolen, z. B. die Handelsprodukte Finsolv® TN (C₁₂-C₁₅-Alkylbenzoat), Finsolv® SB (Isostearylbenzoat) und Finsolv® EB (Ethylhexylbenzoat);
- den C₈-C₂₂-Fettalkoholestern einwertiger oder mehrwertiger C₂-C₇-Hydroxycarbonsäuren, insbesondere den Estern der Glycolsäure, Milchsäure, Äpfelsäure, Weinsäure, Citronensäure und Salicylsäure. Solche Ester auf Basis von linearen C_{12/15}-Alkanolen, z. B. C₁₂-C₁₅-Alkyllactat, und von in 2-Position verzweigten C_{12/13}-Alkanolen, z. B. Di-C₁₂-C₁₃-Alkylmalat, sind unter dem Warenzeichen Cosmacol® von der Firma Nordmann, Rassmann GmbH & Co, Hamburg, zu beziehen, insbesondere die Handelsprodukte Cosmacol® EMI, Cosmacol® ESI und Cosmacol® ETI;
- den Anlagerungsprodukten von Ethylenoxid und/oder Propylenoxid an ein- oder mehrwertige C₃₋₂₀-Alkanole wie Butanol, Butandiol, Myristylalkohol und Stearylalkohol, z. B. PPG-14-Butylether (Ucon Fluid® AP), PPG-9-Butylether (Breox® B25), PPG-10-Butandiol (Macol® 57), PPG-3-Myristylether (Witconol® APM) und PPG-15-Stearylether (Arlamol® E);
- flüssigen Paraffinölen, Isoparaffinölen, z. B. die Handelsprodukte der Permethyl®⁻Serie, insbesondere Isododecan, Isohexadecan und Isoeicosan, und synthetischen Kohlenwasserstoffen wie Polyisobuten oder Polydecene und alicyclischen Kohlenwasserstoffen, z. B. das Handelsprodukt 1,3-Di-(2-ethyl-hexyl)-cyclohexan (Cetiol® S);
- den verzweigten gesättigten oder ungesättigten Fettalkoholen mit 6 - 30 Kohlenstoffatomen. Diese Alkohole werden häufig auch als Guerbet-Alkohole bezeichnet, da sie nach der Guerbet-Reaktion erhältlich sind. Besonders bevorzugte Alkoholöle sind beispielsweise Hexyldecanol (Eutanol® G), Octyldodecanol und 2-Ethylhexylalkohol;
- Mischungen aus Guerbetalkoholen und Guerbetalkoholestern, z. B. das Handelsprodukt Cetiol® PGL (Hexyldecanol und Hexyldecyllaurat).
- den symmetrischen, unsymmetrischen oder cyclischen Estern der Kohlensäure mit Fettalkoholen, beispielsweise Glycerincarbonat, Dicaprylylcarbonat (Cetiol^{®} CC) oder die Ester der DE-OS 197 56 454;
- Triglyceriden von linearen oder verzweigten, gesättigten oder ungesättigten, gegebenenfalls hydroxylierten C₈₋₃₀-Fettsäuren. Besonders geeignet kann die Verwendung natürlicher Öle, z.B. Sojaöl, Baumwollsaatöl, Sonnenblumenöl, Palmöl, Palmkernöl, Leinöl, Mandelöl, Rizinusöl, Maisöl, Olivenöl, Rapsöl, Sesamöl, Distelöl, Weizenkeimöl, Pfirsichkernöl und die flüssigen Anteile des Kokosöls und dergleichen sein. Geeignet sind aber auch synthetische Triglyceridöle, insbesondere Capric/Caprylic Triglycerides, z. B. die Handelsprodukte Myritol^{®} 318, Myritol^{®} 331 (Cognis) oder Miglyol^{®} 812 (Hüls) mit unverzweigten Fettsäureresten sowie Glyceryltrüsostearin und die Handelsprodukte Estol^{®} GTEH 3609 (Uniqema) oder Myritol^{®} GTEH (Cognis) mit verzweigten Fettsäureresten;
- Dicarbonsäureestern von linearen oder verzweigten C₂-C₁₀-Alkanolen, insbesondere Diisopropyladipat, Di-n-butyladipat, Di-(2-ethylhexyl)adipat, Dioctyladipat, Diethyl-/Din-butyl/ Dioctylsebacat, Diisopropylsebacat, Dioctylmalat, Dioctylmaleat, Dicaprylylmaleat, Diisooctytsuccinat, Di-2-ethylhexylsuccinat und Di-(2-hexyldecyl)-succinat;
- Di-n-alkylethern mit insgesamt 12 bis 36, insbesondere 12 bis 24 C-Atomen, z. B. Din-octylether (Cetiol^{®} OE), Di-n- n-Hexyl-n-octylether und n-Octyl-n-decylether.

Die erfindungsgemäßen Wasser-in-Öl-Emulsionen enthalten weiterhin Cetyl PEG/PPG-10/1 Dimethicone als Wasser-in-Öl-Siliconemulgator. Der mindestens eine Wasser-in-Öl-Siliconemulgator ist bevorzugt in einer Menge von 0,5 - 5 Gew.-%, besonders bevorzugt 1,0 - 2,5 Gew.%, außerordentlich bevorzugt 1,5 - 2,0 Gew.-%, jeweils bezogen auf das Gesamtgewicht der Emulsion, enthalten.

Die erfindungsgemäßen W/O-Emulsionen können in einer weiteren besonders bevorzugten Ausführungsform neben den W/O-Siliconemulgatoren weitere siliconfreie W/O-Emulgatoren enthalten. Geeignete W/O-Emulgatoren sind ausgewählt aus Substanzen der allgemeinen Formel A-O-(CHR¹-X-CHR²-O-)ₐ-A', wobei A und A' gleiche oder verschiedene hydrophobe organische Reste darstellen, a eine Zahl von 1 bis 100, vorzugsweise 2 bis 60, insbesondere 5 bis 40 darstellt, X eine Einfachbindung oder die Gruppe >CHOR³ darstellt, R¹ und R² ein Wasserstoffatom oder eine Methylgruppe darstellen und so gewählt werden, dass nicht beide Reste gleichzeitig Methyl darstellen, und R³ ein Wasserstoffatom oder eine verzweigte oder unverzweigte, gesättigte oder ungesättigte Alkyl- oder Acylgruppe mit 1 bis 20 Kohlenstoffatomen darstellt.
Besonders bevorzugt ist es, wenn der siliconfreie W/O-Emulgator oder die siliconfreien W/O- Emulgatoren so gewählt werden, dass die Reste A und A' gewählt sind aus der Gruppe der verzweigten und unverzweigten, gesättigten und ungesättigten Alkyl- und Acylreste und Hydroxyacylreste mit 10 bis 30 Kohlenstoffatomen sowie ferner aus der Gruppe der über Esterfunktionen miteinander verbundenen Hydroxyacylgruppen, nach dem Schema: OOC-R"-CR'H-(OOC-R"-CR'H)_{b}-OOC-R"-CHR', wobei R' gewählt wird aus der Gruppe der verzweigten und unverzweigten Alkylgruppen mit 1 bis 20 Kohlenstoffatomen und R" gewählt wird aus der Gruppe der verzweigten und unverzweigten Alkylengruppen mit 1 bis 20 Kohlenstoffatomen und b Zahlen von 0 bis 200 annehmen kann.
Weitere geeignete siliconfreie W/O-Emulgatoren sind bevorzugt ausgewählt aus
(1) gesättigten Alkoholen mit 8 - 24 C-Atomen, insbesondere mit 16 - 22 C-Atomen, z. B. Cetylalkohol, Stearylalkohol, Arachidylalkohol oder Behenylalkohol oder Gemischen dieser Alkohole, wie sie bei der technischen Hydrierung von pflanzlichen und tierischen Fettsäuren erhalten werden;
(2) ethoxylierten Alkoholen und Carbonsäuren mit 8 - 24 C-Atomen, insbesondere mit 16 - 22 C-Atomen, die einen HLB-Wert von 1 - 8 aufweisen;
(3) propoxylierten Alkoholen und Carbonsäuren mit 8 - 24 C-Atomen, insbesondere mit 16 - 22 C-Atomen;
(4) Partialestern aus einem Polyol mit 3 - 6 C-Atomen und gesättigten und/oder ungesättigten, verzweigten und/oder unverzweigten Fettsäuren mit 8 - 24, insbesondere 12 - 18 C-Atomen. Solche Partialester sind z. B. die Monoglyceride von Palmitin-, Stearinsäure und Ölsäure, die Sorbitanmono- und/oder -diester, insbesondere diejenigen von Myristinsäure, Palmitinsäure, Stearinsäure oder von Mischungen dieser Fettsäuren. Hier sind auch die Monoester aus Trimethylolpropan, Erythrit oder Pentaerythrit und gesättigten Fettsäuren mit 14 - 22 C-Atomen zu nennen. Auch die technischen Monoester, die durch Veresterung von 1 Mol Polyol mit 1 Mol Fettsäure erhalten werden, und ein Gemisch aus Monoester, Diester, Triester und ggf. unverestertem Polyol darstellen, sind einsetzbar.
(5) Polyglycerinestern gesättigter und/oder ungesättigter, verzweigter und/oder unverzweigter Alkancarbonsäuren einer Kettenlänge von 8 - 24, insbesondere 12 - 18 C-Atomen, mit bis zu 10 Glycerineinheiten, vorzugsweise bis zu 3 Glycerineinheiten und einem Verersterungsgrad von 1 - 10, vorzugsweise 1 - 5;
(6) Mono- und/oder Polyglycerinethern gesättigter und/oder ungesättigter, verzweigter und/oder unverzweigter Alkohole einer Kettenlänge von 8 - 30, insbesondere 12 - 18 C-Atomen, mit bis zu 10 Glycerineinheiten, vorzugsweise bis zu 3 Glycerineinheiten und einem Veretherungsgrad von 1 - 10, vorzugsweise 1 - 5;
(7) Propylenglycolestern gesättigter und/oder ungesättigter, verzweigter und/oder unverzweigter Alkancarbonsäuren einer Kettenlänge von 8 - 24, insbesondere 12 - 18 C-Atomen;
(8) Methylglucose-Estern gesättigter und/oder ungesättigter, verzweigter und/oder unverzweigter Alkancarbonsäuren einer Kettenlänge von 8 - 24, insbesondere 12 - 18 C-Atomen;
(9) Polyglycerin-Methylglucose-Estern gesättigter und/oder ungesättigter, verzweigter und/oder unverzweigter Alkancarbonsäuren einer Kettenlänge von 8 - 24, insbesondere 12 - 18 C-Atomen;
(10) Verbindungen der allgemeinen Formel R¹ - O - R², in der R¹ eine primäre lineare Alkyl-, Alkenyl- oder Acylgruppe mit 20 - 30 C-Atomen und R² Wasserstoff, eine Gruppe mit der Formel -(CₙH₂ₙO)ₓ-H mit x = 1 oder 2 und n = 2 - 4 oder eine Polyhydroxyalkylgruppe mit 4 - 6 C-Atomen und 2 - 5 Hydroxylgruppen ist. Weitere bevorzugt geeignete Emulgatoren mit einem HLB-Wert von 8 und darunter sind die Anlagerungsprodukte von 1 oder 2 Mol Ethylenoxid oder Propylenoxid an Behenylalkohol, Erucylalkohol, Arachidylalkohol oder auch an Behensäure oder Erucasäure. Bevorzugt eignen sich auch die Monoester von C₁₆-C₃₀-Fettsäuren mit Polyolen wie z. B. Pentaerythrit, Trimethylolpropan, Diglycerin, Sorbit, Glucose oder Methylglucose. Beispiele für solche Produkte sind z. B. Sorbitanmonobehenat oder Pentaerythrit-monoerucat.
Es kann erfindungsgemäß von Vorteil sein, dass niedrig ethoxylierte (3 - 5 EO) und/oder propoxylierte Produkte Verwendung finden, beispielsweise polyethoxyliertes hydrogeniertes oder nichthydrogeniertes Ricinusöl oder ethoxyliertes Cholesterin.

Besonders bevorzugte zusätzliche siliconfreie W/O-Emulgatoren sind Glyceryllanolat, Glycerylmonostearat, Glyceryldistearat, Glycerylmonoisostearat, Glycerylmonomyristat, Glycerylmonooleat, Diglycerylmonostearat, Glycerylmonolaurat, Glycerylmonocaprinat, Glycerylmonocaprylat, Diglycerylmonoisostearat, Diglyceryldiisostearat, Propylenglycolmonostearat, Propylenglycolmonolaurat, Sorbitanmonoisostearat, Sorbitanmonolaurat, Sorbitanmonocaprylat, Sorbitansesquistearat, Sorbitanmonoisooleat, Saccharosedistearat, Cetylalkohol, Stearylalkohol, Arachidylalkohol, Behenylalkohol, Isobehenylalkohol, 2-Ethylhexylglycerinether, Selachylalkohol, Chimylalkohol, Polyethylenglycol(2)stearylether (Steareth-2), Glycerylsorbitanstearat, Polyglyceryl-4-Isostearat, Polyglyceryl-2-sesquiisostearat, PEG-7-hydrogeniertes Ricinusöl, Isostearyldiglycerylsuccinat, PEG-5-Cholesterylether, PEG-30 Dipolyhydroxystearat, Decaglycerylheptaoleat, Polyglyceryl-3-diisostearat (Handelsprodukt Lameform^{®} TGI), PEG-8 Distearat, Diglycerin Dipolyhydroxystearat (Handelsprodukt Dehymuls^{®} PGPH), Glycerinisostearat, Sorbitanisostearat, Polyglyceryl-3-methylglucosedistearat, polyethoxyliertes hydrogeniertes oder nichthydrogeniertes Ricinusöl, Sterole (Sterine), insbesondere Cholesterol, Lanosterol, Beta-Sitosterol, Stigmasterol, Campesterol und Ergosterol sowie Mykosterole, ethoxyliertes Cholesterin, PEG-2 Stearat, PEG-45/Dodecylglycolcopolymer, PEG-22/Dodecylglycolcopolymer und Methoxy PEG- 22/Dodecyl Glycol Copolymer.

Weitere besonders bevorzugte erfindungsgemäße Zusammensetzungen sind dadurch gekennzeichnet, dass zusätzlich zu dem mindestens einen W/O-Siliconemulgator und dem gegebenenfalls weiteren siliconfreien W/O-Emulgator auch mindestens ein O/W-Emulgator enthalten ist.

Die erfindungsgemäßen Pigmente, die Falten, Fältchen und feine Linien sowie andere Hautunregelmäßigkeiten optisch kaschieren, weisen eine mittlere Partikelgröße von 0,5 - 25 µm, bevorzugt 1-20 µm, besonders bevorzugt 2 - 10 µm und besonders bevorzugt 5 - 7 µm auf. Die Teilchengrößenbestimmung erfolgt mittels Laserlichtstreuung. Im Wellenlängenbereich von 400 - 800 nm zeigen sie diffuse Lichtstreuungseigenschaften und weisen bei 400 - 800 nm einen Transmissionswert von 60 bis mindestens 95 %, bevorzugt 75 bis mindestens 95 % und besonders bevorzugt 85 bis mindestens 95 % auf.
Das mindestens eine Pigment mit einer mittleren Partikelgröße von 0,5 - 25 µm, das im Wellenlängenbereich von 400 - 800 nm diffuse Lichtstreuungseigenschaften und einen Transmissionswert von 60 bis mindestens 95 % aufweist, ist ausgewählt aus sphärischen Kieselsäurepartikeln, die mit Titandioxid (CI 77891) und Eisenoxid (CI 77491) beschichtet sind. Hierunter besonders bevorzugt sind Partikel mit einem SiO₂-Anteil von 74 - 85 Gew.-%, einem TiO₂-Anteil von 15-24 Gew.-% und einem Eisenoxid-Anteil von weniger als 2 Gew.-%, jeweils bezogen auf das Gesamtgewicht des Pigmentes. Besonders bevorzugt ist das Handelsprodukt Ronasphere LDP der Merck KGaA, das eine durchschnittliche Teilchengröße unter 10 µm aufweist.
Weiterhin ist das mindestens eine Pigment mit einer mittleren Partikelgröße von 0,5 - 25 µm, das im Wellenlängenbereich von 400 - 800 nm diffuse Lichtstreuungseigenschaften und einen Transmissionswert von 60 bis mindestens 95 % aufweist, ausgewählt aus sphärischen Partikeln aus vernetztem Polymethylmethacrylat mit der INCI-Bezeichnung Methyl Methacrylate Cross Polymer. Besonders bevorzugt sind derartige Partikel mit einer durchschnittlichen Teilchengröße von 5-10 µm, wobei das Handelsprodukt SUNPMMA-S der Firma Sunjin Chemical Co. ganz besonders bevorzugt ist.
Weiterhin ist das mindestens eine Pigment mit einer mittleren Partikelgröße von 0,5 - 25 µm, das im Wellenlängenbereich von 400 - 800 nm diffuse Lichtstreuungseigenschaften und einen Transmissionswert von 60 bis mindestens 95 % aufweist, ausgewählt aus sphärischen TiO₂-hattigen Kieselsäurepartikeln. Besonders bevorzugt sind derartige Partikel mit einer durchschnittlichen Teilchengröße von 2-7 µm, wobei die Handelsprodukte SUNSIL Tin (Kieselsäure-verkapseltes Titandioxid) der Firma Sunjin Chemical Co., insbesondere SUNSIL Tin 30, ganz besonders bevorzugt sind. Weiterhin ist es erfindungsgemäß besonders bevorzugt, beliebige Mischungen der vorgenannten Pigmente einzusetzen.
Das mindestens eine Pigment mit einer mittleren Partikelgröße von 0,5 - 25 µm, das im Wellenlängenbereich von 400 - 800 nm diffuse Lichtstreuungseigenschaften und einen Transmissionswert von 60 bis mindestens 95% aufweist, ist in den erfindungsgemäßen Zusammensetzungen in einer Menge von 0,5 - 7 Gew.%, besonders bevorzugt 1 - 4 Gew.-% und außerordentlich bevorzugt 2-3 Gew.-%, jeweils bezogen auf die Gesamtzusammensetzung, enthalten.

Um die pflegende Wirkung der erfindungsgemäßen Zusammensetzungen noch zu erhöhen, sind in einer bevorzugten Ausführungsform der Erfindung weitere kosmetische Wirkstoffe enthalten.

Überraschend wurde festgestellt, dass ein Gehalt an Feuchthaltemitteln den optischen Kaschiereffekt der erfindungsgemäßen Zusammensetzungen weiter in unerwartet hohem Ausmaß steigern kann. Besonders bevorzugte erfindungsgemäße Zusammensetzungen sind daher dadurch gekennzeichnet, dass mindestens ein Feuchthaltemittel, insbesondere ausgewählt aus den wasserlöslichen mehrwertigen C₂ - C₉-Alkanolen mit 2 - 6 Hydroxylgruppen und/oder den wasserlöslichen Polyethylenglycolen mit 3 - 20 Ethylenoxid-Einheiten sowie Mischungen hiervon, enthalten ist. Bevorzugt sind diese Komponenten ausgewählt aus 1,2-Propylenglycol, 2-Methyl-1,3-propandiol, Glycerin, Butylenglycolen wie 1,2-Butylenglycol, 1,3-Butylenglycol und 1,4-Butylenglycol, Pentylenglycolen, Hexandiolen wie 1,6-Hexandiol, Hexantriolen wie 1,2,6-Hexantriol, 1,8-Octandiol, Dipropylenglycol, Tripropylenglycol, Diglycerin, Triglycerin, Erythrit, Sorbit sowie Mischungen der vorgenannten Substanzen. Geeignete wasserlösliche Polyethylenglycole sind ausgewählt aus PEG-3, PEG-4, PEG-6, PEG-7, PEG-8, PEG-9, PEG-10, PEG-12, PEG-14, PEG-16, PEG-18 und PEG-20 sowie Mischungen hiervon, wobei PEG-3 bis PEG-8 bevorzugt sind. Auch Zucker und bestimmte Zuckerderivate wie Fructose, Glucose, Maltose, Maltitol, Mannit, Inosit, Sucrose, Trehalose und Xylose sind erfindungsgemäß geeignet. Weitere bevorzugte Feuchthaltemittel sind (2-Hydroxyethyl) - harnstoff und Glycosaminoglycane und deren Salze und/oder Ester, insbesondere Hyaluronsäure, ihre Salze und ihre Silanolderivate.
Die erfindungsgemäßen Zusammensetzungen können bevorzugt mindestens ein Feuchthaltemittel in Mengen von 0,5 - 25 Gew.-%, bevorzugt 2 - 15 Gew.-%, besonders bevorzugt 5 - 10 Gew.-%, bezogen auf die Gesamtzusammensetzung, enthalten.

Weitere bevorzugte Wirkstoffe, die ebenfalls den optischen Kaschiereffekt der erfindungsgemäßen Zusammensetzungen in unerwartet hohem Ausmaß steigern können, sind ausgewählt aus Monomeren, Oligomeren und Polymeren von Aminosäuren, N-C₂₋C₂₄-Acylaminosäuren, den Estern und/oder den physiologisch verträglichen Metallsalzen dieser Substanzen, DNA- oder RNA-Oligonucleotiden, natürlichen Betainverbindungen, Vitaminen, Provitaminen und Vitaminvorstufen der Gruppen A, B, C, E, H und K und den Estern der vorgenannten Substanzen, α-Hydroxycarbonsäuren, α-Ketocarbonsäuren, β-Hydroxycarbonsäuren und deren Ester-, Lacton- oder Salzform, Flavonoiden und Flavonoid-reichen Pflanzenextrakten, Isoflavonoiden und Isoflavonoid-reichen Pflanzenextrakten, Polyphenolen und Polyphenol-reichen Pflanzenextrakten, Ubichinon und Ubichinol sowie deren Derivaten, Silymarin, natürlich vorkommenden Xanthin-Derivaten, ausgewählt aus Coffein, Theophyllin, Theobromin und Aminophyllin, Ectoin, anorganischen und organischen UV-Filtersubstanzen, selbstbräunenden Wirkstoffen, hautaufhellenden Wirkstoffen, hautberuhigenden Wirkstoffen, sowie Desoxyzuckern oder Desoxyzucker-Bausteine enthaltenden Polysacchariden.

Die Monomere der Aminosäuren und/oder der N-C₂-C₂₄-Acylaminosäuren sind ausgewählt aus Alanin, Arginin, Asparagin, Asparaginsäure, Canavanin, Citrullin, Cystein, Cystin, Dipalmitoylhydroxyprolin, Desmosin, Glutamin, Glutaminsäure, Glycin, Histidin, Homophenylalanin, Hydroxylysin, Hydroxyprolin, Isodesmosin, Isoleucin, Leucin, Lysin, Methionin, Methylnorleucin, Ornithin, Phenylalanin, Prolin, Pyroglutaminsäure, Sarcosin, Serin, Taurin, Threonin, Thyroxin, Tryptophan, Tyrosin, Valin, N-Acetyl-L-cystein, Zinkpyroglutamat, Natriumoctanoylglutamat, Natriumdecanoylglutamat, Natriumlauroylglutamat, Natriummyristoylglutamat, Natriumcetoylglutamat und Natriumstearoylglutamat. Besonders bevorzugt sind Lysin, Serin, Zink- und Natriumpyroglutamat und Natriumlauroylglutamat.
Der C₂ - C₂₄-Acylrest, mit dem die genannten Aminosäuren an der Aminogruppe derivatisiert sind, ist ausgewählt aus einem Acetyl-, Propanoyl-, Butanoyl-, Pentanoyl-, Hexanoyl-, Heptanoyl-, Octanoyl-, Nonanoyl-, Decanoyl-, Undecanoyl-, Lauroyl-, Tridecanoyl-, Myristoyl-, Pentadecanoyl-, Cetoyl-, Palmitoyl-, Stearoyl-, Elaidoyl-, Arachidoyl- oder Behenoyl-Rest. Mischungen von C₈-C₁₈-Acylresten werden auch als Cocoyl-Rest bezeichnet und sind ebenfalls bevorzugte Substituenten.
Mit den vorgenannten C₂ - C₂₄-Acylresten können die Aminosäuren, die eine OH-Gruppe tragen, auch an dieser OH-Gruppe verestert sein. Ein erfindungsgemäß bevorzugtes Beispiel hierfür ist Hydroxyprolin, das mit zwei, bevorzugt linearen, C₂-C₂₂-Fettsäureresten N-acyliert und verestert ist, besonders bevorzugt Dipalmitoylhydroxyprolin, das z. B. unter der Bezeichnung Sepilift DPHP von der Firma Seppic erhältlich ist.
Die physiologisch verträglichen Salze der erfindungsgemäß bevorzugten Aminosäuren oder Aminosäurederivate sind bevorzugt ausgewählt aus den Ammonium-, Alkalimetall-, Magnesium-, Calcium-, Aluminium-, Zink- und Mangan-Salzen. Besonders bevorzugt sind die Natrium-, Kalium-, Magnesium-, Aluminium-, Zink- und Mangan-Salze.
Unter Aminosäureoligomeren werden erfindungsgemäß Peptide mit 2 - 30, bevorzugt 2 - 15, Aminosäuren, verstanden. Die Oligomere der Aminosäuren und/oder der N-C₂-C₂₄₋Acylaminosäuren sind bevorzugt ausgewählt aus Di-, Tri-, Tetra-, Penta-, Hexa- oder Pentadecapeptiden, die N-acyliert und/oder verestert sein können. Zahlreiche dieser Aminosäureoligomere stimulieren die Collagensynthese beziehungsweise sind in der Lage, Zellen des Immunsystems, wie Mastzellen und Makrophagen, zu rekrutieren, die dann über die Freisetzung von Wachstumsfaktoren Reparaturprozesse im Gewebe, z.B. die Collagensynthese, induzieren, beziehungsweise sind in der Lage, an die Sequenz Arg-Phe-Lys in Thrombospondin I (TSP-1) zu binden und damit aktives TGF-β (*tissue growth factor*), der die Synthese von Collagen in dermalen Fibroblasten induziert, freizusetzen. Derartige Aminosäureoligomere werden bevorzugt als Wirkstoffe gegen die Hautalterung verwendet.
Erfindungsgemäß bevorzugte, gegebenenfalls N-acylierte und/oder veresterte Dipeptide sind Acetyl-Citrullyl-Arginin (z. B. Exsy-Algine von Exsymol mit der INCI-Bezeichnung Acetyl Citrull Amido Arginine), Tyr-Arg (Dipeptide-1), Val-Trp (Dipeptide-2), Asn-Phe, Asp-Phe, N-Palmitoyl-β-Ala-His, N-Acetyl-Tyr-Arg-hexyldecylester (z. B. Calmosensine von Sederma), Carnosin (β-Ala-His) und N-Palmitoyl-Pro-Arg. Erfindungsgemäß bevorzugte, gegebenenfalls N-acylierte und/oder veresterte Tripeptide sind Gly-His-Lys, das z. B. unter der Bezeichnung "Omega-CH-Aktivator" von der Firma GfN oder in acylierter Form (N-Palmitoyl-Gly-His-Lys) unter der Bezeichnung Biopeptide CL von Sederma erhältlich ist, aber (in acylierter Form) auch einen Bestandteil des Produktes Matrixyl 3000 von Sederma darstellt. Das Tripeptid Gly-His-Lys kann auch als Kupfersalz (Cu²⁺) eingesetzt werden und ist als solches über ProCyte Corporation zu beziehen. Weiterhin können Analoga von Gly-His-Lys eingesetzt werden, wobei maximal zwei Aminosäuren durch geeignete andere Aminosäuren substituiert sind. Zur Substitution von Gly sind erfindungsgemäß Ala, Leu und Ile geeignet. Die erfindungsgemäß bevorzugten Aminosäuren, die His oder Lys ersetzen können, beinhalten eine Seitenkette mit einem Stickstoffatom, das bei pH 6 überwiegend geladen vorliegt, z. B. Pro, Lys, Arg, His, Desmosin und Isodesmosin. Besonders bevorzugt wird Lys durch Arg, Orn, oder Citrullin ersetzt. Ein weiteres erfindungsgemäß bevorzugtes Tripeptid ist Gly-His-Arg (INCI-Bezeichnung: Tripeptide-3) sowie dessen Derivat N-Myristoyl-Gly-His-Arg, das z. B. unter der Bezeichnung Collasyn 314-GR von Therapeutic Peptide Inc. erhältlich ist; weitere erfindungsgemäß bevorzugte Tripeptide sind ausgewählt aus Lys-Val-Lys, Lys-Val-Dab (Dab = Diaminobuttersäure), Lys-Phe-Lys, Lys-Ile-Lys, Dab-Val-Lys, Lys-Val-Orn, Lys-Val-Dap (Dap = Diaminopropionsäure), Dap-Val-Lys, Palmitoyl-Lys-Val-Lys, z. B. erhältlich von der Firma Pentapharm unter der Bezeichnung SYN® -COLL, Lys-Pro-Val, Tyr-Tyr-Val, Tyr-Val-Tyr, Val-Tyr-Val (Tripeptide-2), Tripeptide-4 (z. B. ATPeptide, zu beziehen über IMPAG), His-Ala-Orn N-Elaidoyl-Lys-Phe-Lys und N-Acetyl-Arg-Lys-Arg-NH₂.
Erfindungsgemäß bevorzugte, gegebenenfalls N-acylierte und/oder veresterte Tetrapeptide sind ausgewählt aus Rigin und Rigin-basierten Tetrapeptiden sowie ALAMCAT-Tetrapeptiden. Rigin weist die Sequenz Gly-Gln-Pro-Arg auf. Rigin-basierte Tetrapeptide umfassen die Rigin-Analoga und Rigin-Derivate, insbesondere das erfindungsgemäß besonders bevorzugte N-Palmitoyl-Gly-Gln-Pro-Arg, das z. B. unter der Bezeichnung Eyeliss von Sederma erhältlich ist, aber auch einen Bestandteil des Produktes Matrixyl 3000 von Sederma darstellt. Zu den Rigin-Analoga zählen solche, bei denen die vier Aminosäuren umarrangiert sind und/oder bei denen gegenüber Rigin maximal zwei Aminosäuren substituiert sind, z. B. die Sequenz Ala-Gln-Thr-Arg. Bevorzugt hat mindestens eine der Aminosäuren der Sequenz ein Pro oder Arg und besonders bevorzugt beinhaltet das Tetrapeptid sowohl Pro als auch Arg, wobei ihre Reihenfolge und Position variieren können. Die substituierenden Aminosäuren können aus jeder Aminosäure, die im folgenden definiert ist, ausgewählt werden. Besonders bevorzugte Rigin-basierte Tetrapetide umfassen: Xaa-Xbb-Arg-Xcc, Xaa-Xbb-Xcc-Pro, Xaa-Xbb-Pro-Arg, Xaa-Xbb-Pro-Xcc, Xaa-Xbb-Xcc-Arg, wobei Xaa, Xbb und Xcc gleiche oder voneinander verschiedene Aminosäuren sein können und wobei Xaa ausgewählt ist aus Gly und den Aminosäuren, die Gly substituieren können, Xbb ausgewählt ist aus Gln und den Aminosäuren, die Gln substituieren können, Xcc ausgewählt ist aus Pro oder Arg und den Aminosäuren, die Pro und Arg substituieren können.
Die bevorzugten Aminosäuren, die Gly ersetzen können, beinhalten eine aliphatische Seitenkette, z. B. β-Ala, Ala, Val, Leu, Pro, Sarcosin (Sar) und Isoleucin (Ile).
Die bevorzugten Aminosäuren, die Gln ersetzen können, beinhalten eine Seitenkette mit einer Aminogruppe, die bei neutralem pH (pH 6-7) überwiegend ungeladen vorliegt, z.B. Asn, Lys, Orn, 5-Hydroxyprolin, Citrullin und Canavanin.
Die bevorzugten Aminosäuren, die Arg ersetzen können, beinhalten eine Seitenkette mit einem Stickstoffatom, das bei pH 6 überwiegend geladen vorliegt, z.B. Pro, Lys, His, Desmosin und Isodesmosin.
Als Rigin-Analoga sind Gly-Gln-Arg-Pro und Val-Val-Arg-Pro erfindungsgemäß bevorzugt.
ALAMCAT-Tetrapeptide sind Tetrapeptide, die mindestens eine Aminosäure mit einer aliphatischen Seitenkette enthalten, z. B. β-Ala, Ala, Val, Leu, Pro, Sarcosin (Sar) und Isoleucin (Ile). Weiterhin beinhalten ALAMCAT-Tetrapeptide mindestens eine Aminosäure mit einer Seitenkette mit einer Aminogruppe, die bei neutralem pH (pH 6-7) überwiegend ungeladen vorliegt, z.B. Gln, Asn, Lys, Orn, 5-Hydroxyprolin, Citrullin und Canavanin. Weiterhin beinhalten ALAMCAT-Tetrapeptide mindestens eine Aminosäure mit einer Seitenkette mit einem Stickstoffatom, das bei pH 6 überwiegend geladen vorliegt, z. B. Arg, Pro, Lys, His, Desmosin und Isodesmosin. Als vierte Aminosäure können ALAMCAT-Tetrapeptide jede beliebige Aminosäure enthalten; bevorzugt ist jedoch auch die vierte Aminosäure aus den drei vorstehend genannten Gruppen ausgewählt.
Erfindungsgemäß bevorzugte, gegebenenfalls N-acylierte und/oder veresterte Pentapeptide sind ausgewählt aus Lys-Thr-Thr-Lys-Ser und seinen N-acylierten Derivaten, besonders bevorzugt N-Palmitoyl-Lys-Thr-Thr-Lys-Ser, das unter der Bezeichnung Matrixyl von der Firma Sederma erhältlich ist, weiterhin N-Palmitoyl-Tyr-Gly-Gly-Phe-Met, Val-Val-Arg-Pro-Pro, N-Palmitoyl-Tyr-Gly-Gly-Phe-Leu, Gly-Pro-Phe-Pro-Leu und N-Benzyloxycarbonyl-Gly-Pro-Phe-Pro-Leu (die beiden letztgenannten stellen Serinproteinase-Inhibitoren zur Inhibition der Desquamation dar). Erfindungsgemäß bevorzugte, gegebenenfalls N-acylierte und/oder veresterte Hexapeptide sind Val-Gly-Val-Ala-Pro-Gly und seine N-acylierten Derivate, besonders bevorzugt N-Palmitoyl-Val-Gly-Val-Ala-Pro-Gly, das unter der Bezeichnung Biopeptide EL von der Firma Sederma erhältlich ist, weiterhin Acetyl-Hexapeptide-3 (Argireline von Lipotec), Hexapeptide-4 (z. B. Collasyn 6KS von Therapeutic Peptide Inc. (TPI)), Hexapeptide-5 (z. B. Collasyn 6VY von TPI), Myristoyl Hexapeptide-5 (z. B. Collasyn 614VY von TPI), Myristoyl Hexapeptide-6 (z. B. Collasyn 614VG von TPI), Hexapeptide-8 (z. B. Collasyn 6KS von TPI), Myristoyl Hexapeptide-8 (z. B. Collasyn Lipo-6KS von TPI), Hexapeptide-9 (z. B. Collaxyl von Vincience) und Hexapeptide-10 (z. B. Collaxyl von Vincience oder Seriseline von Lipotec), Ala-Arg-His-Leu-Phe-Trp (Hexapeptide-1), Acetyl Hexapeptide-1 (z. B. Modulene von Vincience), Acetyl Glutamyl Hexapeptide-1 (z. B. SNAP-7 von Centerchem), Hexapeptide-2 (z. B. Melanostatine-DM von Vincience), Ala-Asp-Leu-Lys-Pro-Thr (Hexapeptide-3, z. B. Peptide 02 von Vincience), Val-Val-Arg-Pro-Pro-Pro, Hexapeptide-4 (z. B. Collasyn 6KS von Therapeutic Peptide Inc. (TPI)), Hexapeptide-5 (z. B. Collasyn 6VY von TPI), Myristoyl Hexapeptide-5 (z. B. Collasyn 614VY von TPI), Myristoyl Hexapeptide-6 (z. B. Collasyn 614VG von TPI), Ala-Arg-His-Methylnorleucin-Homophenylalanin-Trp (Hexapeptide-7), Hexapeptide-8 (z. B. Collasyn 6KS von TPI), Myristoyl Hexapeptide-8 (z. B. Collasyn Lipo-6KS von TPI), Hexapeptide-9 (z. B. Collaxyl von Vincience), Hexapeptide-10 (z. B. Collaxyl von Vincience oder Seriseline von Lipotec) und Hexapeptide-11 (z. B. Peptamide-6 von Arch Personal Care). Ein erfindungsgemäß bevorzugtes Pentadecapeptid ist z. B. der Rohstoff Vinci 01 von Vincience (Pentadecapeptide-1). Ein weiteres bevorzugtes Aminosäureoligomer ist das Peptidderivat L-Glutamylaminoethyl-indol (Glistin von Exsymol).
Erfindungsgemäß besonders bevorzugt ist die Kombination aus N-Palmitoyl-Gly-His-Lys und N-Palmitoyl-Gly-Gln-Pro-Arg, wie sie beispielsweise in dem Rohstoff Matrixyl 3000 von der Firma Sederma erhältlich ist.
Es kann erfindungsgemäß besonders bevorzugt sein, ein Gemisch aus mindestens zwei Oligopeptiden einzusetzen. Ein besonders bevorzugtes Gemisch ist die Kombination aus N-Palmitoyl-Gly-His-Lys (z.B. Biopeptide CL von Sederma) und N-Palmitoyl-Gly-Gln-Pro-Arg (z.B. in Eyeliss von Sederma). Eine vorgefertigte Mischung des Tripeptids Palmitoyl-Gly-His-Lys und des Tetrapeptids N-Palmitoyl-Gly-Gln-Pro-Arg ist unter dem Handelsnamen Matrixyl 3000, ebenfalls von Sederma, erhältlich.

Die Polymere der Aminosäuren und/oder der N-C₂-C₂₄-Acylaminosäuren sind ausgewählt aus pflanzlichen und tierischen Proteinhydrolysaten und/oder Proteinen mit mehr als 30 Aminosäurebausteinen. Tierische Proteinhydrolysate sind z. B. Elastin-, Collagen-, Keratin-, Seiden- und Milcheiweiß-Proteinhydrolysate, die auch in Form von Salzen vorliegen können. Erfindungsgemäß bevorzugt sind pflanzliche Proteinhydrolysate, z. B. Soja-, Weizen-, Mandel-, Erbsen-, Kartoffel- und Reisproteinhydrolysate. Entsprechende Handelsprodukte sind z. B. DiaMin® (Diamalt), Gluadin® (Cognis), Lexein® (Inolex) und Crotein® (Croda). Besonders bevorzugt sind Sojaproteinhydrolysate, z. B. die Handelsprodukte Phytokine von Coletica oder Ridulisse C von Silab.
Proteinhydrolysate können naturgemäß auch monomere Aminosäuren und Oligopeptide enthalten; ihre Zusammensetzung ist normalerweise nicht definiert.
Ebenfalls möglich ist der Einsatz von Acylderivaten der Proteinhydrolysate, z. B. in Form ihrer Fettsäure-Kondensationsprodukte. Entsprechende Handelsprodukte sind z. B. Lamepon® (Cognis), Gluadin® (Cognis), Lexein® (Inolex), Crolastin® oder Crotein® (Croda).
Erfindungsgemäß einsetzbar sind auch kationisierte Proteinhydrolysate. Bevorzugt sind kationische Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Weiterhin können die kationischen Proteinhydrolysate auch noch weiter derivatisiert sein. Als typische Beispiele für erfindungsgemäß verwendete kationische Proteinhydrolysate und -derivate sind einige der unter den INCI- Bezeichnungen im "International Cosmetic Ingredient Dictionary and Handbook", (seventh edition 1997, The Cosmetic, Toiletry, and Fragrance Association 1101 17^{th} Street, N.W., Suite 300, Washington, DC 20036-4702) genannten und im Handel erhältlichen Produkte aufgeführt: Cocodimonium Hydroxypropyl Hydrolyzed Collagen, Steardimonium Hydroxypropyl Hydrolyzed Collagen, Cocodimonium Hydroxypropyl Hydrolyzed Rice Protein, Cocodimonium Hydroxypropyl Hydrolyzed Silk, Cocodimonium Hydroxypropyl Hydrolyzed Soy Protein, Cocodimonium Hydroxypropyl Hydrolyzed Wheat Protein, Cocodimonium Hydroxypropyl Silk Amino Acids, Hydroxypropyl Arginine Lauryl/Myristyl Ether HCl. Ganz besonders bevorzugt sind die kationischen Proteinhydrolysate und -derivate auf pflanzlicher Basis.
In einer weiteren bevorzugten Ausführungsform der Erfindung sind die Polymeren der Aminosäuren ausgewählt aus DNA-Reparaturenzymen.
Erfindungsgemäß besonders bevorzugte DNA-Reparaturenzyme sind Photolyase und T4 Endonuclease V, letztere im weiteren mit "T4N5" abgekürzt. Diese beiden Enzyme sind im Stand der Technik bereits als sogenannte DNA-Reparatur-Enzyme bekannt. Unter DNA-Reparatur ist definitionsgemäß die Spaltung bzw. Entfernung von UV-induzierten Pyrimidindimeren aus der DNA zu verstehen.
Photolyase ist die Kurzbezeichnung für Desoxyribodipyrimidin-Photolyase bzw. DNA-Photolyase, ein Enzym mit der Klassifizierungsnummer EC 4.1.99.3. Eine besonders effiziente Photolyase stammt aus *Anacystis nidulans*, einem phototrophen marinen Mikroorganismus. Die Photolyase aus *A. nidulans* wird in technisch relevanten Mengen mittlerweile aus E. coli gewonnen. Photolyase ist zur Aktivierung auf Licht angewiesen.
Das Enzym T4 Endonuclease V wird vom denV-Gen der Bakteriophage T4 produziert und gehört zu den Phosphodiesterasen, die die Nucleinsäuren an der (5'-3')-Bindung hydrolytisch spalten. T4N5 ist auch ohne Lichteinfluss aktiv.
Erfindungsgemäß besonders bevorzugt ist der Einsatz von liposomenverkapselten DNA-Reparaturenzymen. Liposomenverkapselte Photolyase ist im Handel z. B. unter der Produktbezeichnung Photosome™, liposomenverkapselte T4N5 z. B. unter der Bezeichnung Ultrasome™ von der Firma AGI Dermatics, USA, erhältlich.
Besonders bevorzugte erfindungsgemäße Zusammensetzungen enthalten Photosome™ und/oder Ultrasome™ in Gesamtmengen von 0,1 - 10 Gew.-%, bevorzugt 0,5 - 5,0 Gew.-% und besonders bevorzugt 1,0 - 4,0 Gew.-%, bezogen auf die gesamte Zusammensetzung.
Besonders bevorzugte erfindungsgemäße Zusammensetzungen sind dadurch gekennzeichnet, dass sie die Monomere, Oligomere oder Polymere von Aminosäuren, N-C₂-C₂₄₋Acylaminosäuren und/oder C₂-C₂₄-Aminosäureestern und/oder den physiologisch verträglichen Salzen dieser Substanzen in einer Gesamtmenge von 0,00001 - 10 Gew.-%, bevorzugt 0,001 - 5 Gew.-% und besonders bevorzugt 0,005 - 3 Gew.-%, jeweils bezogen auf die gesamte Zusammensetzung, enthalten.

In einer weiteren bevorzugten Ausführungsform enthalten die erfindungsgemäßen Zusammensetzungen mindestens ein DNA-Oligonucleotid oder ein RNA-Oligonucleotid. Erfindungsgemäß werden unter einem Oligonucleotid Polymerisate aus 2 bis 20, bevorzugt 2 bis 10 Mononucleotiden verstanden, die ebenso wie bei Polynucleotiden und Nucleinsäuren durch Phosphorsäurediester-Brücken verknüpft sind. Die Nucleotide bestehen aus Nucleobasen (meist Pyrimidin- oder Purin-Derivaten), Pentosen (meist D-Ribofuranose oder 2-Desoxy-D-ribofuranose in β-N-glykosidischer Bindung an die Nucleobase) und Phosphorsäure. Die Mononucleotide sind zum Beispiel Adenosinphosphate, Cytidinphosphate, Guanosinphosphate, Uridinphosphate und Thymidinphosphate, insbesondere CMP (Cytidin-5'-monophosphat), UDP (Uridin-5'-diphosphat), ATP (Adenosin-5 '-triphosphat) und GTP (Guanosin-5'-triphosphat). Ein erfindungsgemäß besonders bevorzugtes Oligonucleotid ist das Thymidin-Dinucleotid.
In den erfindungsgemäßen Zusammensetzungen sind die DNA-Oligonucleotide oder RNA-Oligonucleotide bevorzugt in Mengen von 0,0001 - 5 Gew.-%, besonders bevorzugt 0,001 - 1,0 Gew.-% und außerordentlich bevorzugt 0,01 - 0,5 Gew.-%, bezogen auf die gesamte Zusammensetzungen, enthalten.

Erfindungsgemäß bevorzugte natürliche Betainverbindungen sind natürlich vorkommende Verbindungen mit der Atomgruppierung R₃N⁺-CH₂-X-COO⁻ gemäß IUPAC-Regel C-816.1. Sogenannte Betaintenside (synthetisch) fallen nicht unter die erfindungsgemäß verwendeten Betainverbindungen, ebenso wenig andere zwitterionische Verbindungen, in denen sich die positive Ladung an N oder P und die negative Ladung formal an O, S, B oder C befindet, die aber nicht der IUPAC-Regel C-816.1 entsprechen. Erfindungsgemäß bevorzugte Betainverbindungen sind Betain (Me₃N⁺-CH₂-COO⁻) und Carnitin (Me₃N⁺-CH₂-CHOH-CH₂-COO⁻), jeweils mit Me = Methyl.
Die Betainverbindungen sind in den erfindungsgemäßen W/O-Emulsionen bevorzugt in einer Gesamtmenge von 0,05 bis 5 Gew.-%, besonders bevorzugt 0,1 bis 3 Gew.-%, und außerordentlich bevorzugt 0,5 bis 2 Gew.-%, jeweils bezogen auf die gesamte Zusammensetzung, enthalten.

In einer weiteren bevorzugten Ausführungsform enthalten die erfindungsgemäßen Zusammensetzungen mindestens ein Vitamin, Provitamin oder eine als Vitaminvorstufe bezeichnete Verbindung aus den Vitamingruppen A, B, C, E, H und K und den Estern der vorgenannten Substanzen.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester, wie Retinylpalmitat und Retinylacetat in Betracht. Die erfindungsgemäßen W/O-Emulsionen enthalten die Vitamin A-Komponente bevorzugt in Mengen von 0,05 - 1 Gew.-%, bezogen auf die gesamte Zusammensetzung.

Zur Vitamin B-Gruppe oder zu dem Vitamin B-Komplex gehören unter anderem
- Vitamin B₁, Trivialname Thiamin, chemische Bezeichnung 3-[(4'-Amino-2'-methyl-5'-pyrimidinyl)-methyl]-5-(2-hydroxyethyl)-4-methylthiazoliumchlorid. Bevorzugt wird Thiaminhydrochlorid in Mengen von 0,05 bis 1 Gew.-%, bezogen auf die gesamte W/O-Emulsion, eingesetzt.
- Vitamin B₂, Trivialname Riboflavin, chemische Bezeichnung 7,8-Dimethyl-10-(1-D-ribityl)-benzo[g]pteridin-2,4(3*H*,10*H*)-dion. Bevorzugt werden Riboflavin oder seine Derivate in Mengen von 0,05 bis 1 Gew.-%, bezogen auf die gesamte W/O-Emulsion, eingesetzt.
- Vitamin B₃. Unter dieser Bezeichnung werden die Verbindungen Nicotinsäure und Nicotinsäureamid (Niacinamid) geführt. Erfindungsgemäß bevorzugt ist das Nicotinsäureamid, das in den erfindungsgemäßen Mitteln bevorzugt in Mengen von 0,05 bis 1 Gew.-%, bezogen auf die gesamte W/O-Emulsion, enthalten ist.
- Vitamin B₅ (Pantothensäure und Panthenol). Bevorzugt wird Panthenol eingesetzt. Erfindungsgemäß einsetzbare Derivate des Panthenols sind insbesondere die Ester und Ether des Panthenols sowie kationisch derivatisierte Panthenole. In einer weiteren bevorzugten Ausführungsform der Erfindung können an Stelle von sowie zusätzlich zu Pantothensäure oder Panthenol auch Derivate des 2-Furanon mit der allgemeinen Strukturformel (I) eingesetzt werden.

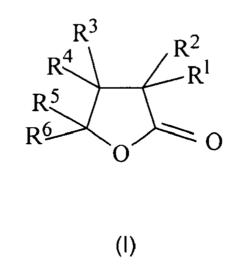

Bevorzugt sind die 2-Furanon-Derivate, in denen die Substituenten R¹ bis R⁶ unabhängig voneinander ein Wasserstoffatom, einen Hydroxylrest, einen Methyl-, Methoxy-, Aminomethyl- oder Hydroxymethylrest, einen gesättigten oder ein- oder zweifach ungesättigten, linearen oder verzweigten C₂-C₄ - Kohlenwasserstoffrest, einen gesättigten oder ein- oder zweifach ungesättigten, verzweigten oder linearen Mono-, Di- oder Trihydroxy-C₂-C₄ - Kohlenwasserstoffrest oder einen gesättigten oder ein- oder zweifach ungesättigten, verzweigten oder linearen Mono-, Di- oder Triamino-C₂-C₄ - Kohlenwasserstoffrest darstellen. Besonders bevorzugte Derivate sind die auch im Handel erhältlichen Substanzen Dihydro-3-hydroxy-4,4-dimethyl-2(3H)-furanon mit dem TriviaInamen Pantolacton (Merck), 4-Hydroxymethyl-γ-butyrolacton (Merck), 3,3-Dimethyl-2-hydroxy-γ-butyrolacton (Aldrich) und 2,5-Dihydro-5-methoxy-2-furanon (Merck), wobei ausdrücklich alle Stereoisomeren eingeschlossen sind. Das erfindungsgemäß außerordentlich bevorzugte 2-Furanon-Derivat ist Pantolacton (Dihydro-3-hydroxy-4,4-dimethyl-2(3H)-furanon), wobei in Formel (I) R¹ für eine Hydroxylgruppe, R² für ein Wasserstoffatom, R³ und R⁴ für eine Methylgruppe und R⁵ und R⁶ für ein Wasserstoffatom stehen. Das Stereoisomer (R)-Pantolacton entsteht beim Abbau von Pantothensäure.
Die genannten Verbindungen des Vitamin B₅-Typs sowie die 2-Furanonderivate sind in den erfindungsgemäßen Mitteln bevorzugt in einer Gesamtmenge von 0,05 bis 5 Gew.-%, besonders bevorzugt 0,1 bis 3 Gew.-%, außerordentlich bevorzugt 0,5 bis 2 Gew.-%, jeweils bezogen auf die gesamte W/O-Emulsion, enthalten.
- Vitamin B₆, wobei man hierunter keine einheitliche Substanz, sondern die unter den Trivialnamen Pyridoxin, Pyridoxamin und Pyridoxal bekannten Derivate des 5-Hydroxymethyl-2-methylpyridin-3-ols versteht. Vitamin B₆ ist in den erfindungsgemäßen W/O-Emulsionen bevorzugt in Mengen von 0,0001 bis 1,0 Gew.-%, insbesondere in Mengen von 0,001 bis 0,01 Gew.-%, enthalten.
- Vitamin B₇ (Biotin), auch als Vitamin H oder "Hautvitamin" bezeichnet. Bei Biotin handelt es sich um (3a*S*,4*S*, 6a*R*)-2-Oxohexahydrothienol[3,4-*d*]-imidazol-4-valeriansäure. Biotin ist in den erfindungsgemäßen W/O-Emulsionen bevorzugt in Mengen von 0,0001 bis 1,0 Gew.-%, insbesondere in Mengen von 0,001 bis 0,01 Gew.-% enthalten.

Vitamin C (Ascorbinsäure) wird bevorzugt in Mengen von 0,1 bis 3 Gew.-%, bezogen auf die gesamte Zusammensetzung, eingesetzt. Die Verwendung der Derivate Ascorbylpalmitat, -stearat, -dipalmitat, -acetat, Mg-Ascorbylphosphat, Na-Ascorbylphosphat, Natrium- und Magnesiumascorbat, Dinatriumascorbylphosphat und -sulfat, Kaliumascorbyltocopherylphosphat, Chitosanascorbat oder Ascorbylglucosid kann bevorzugt sein. Die Verwendung in Kombination mit Tocopherolen kann ebenfalls bevorzugt sein. Zur Vitamin E-Gruppe zählen Tocopherol, insbesondere α-Tocopherol, und seine Derivate. Bevorzugte Derivate sind insbesondere die Ester, wie Tocopherylacetat, -nicotinat, -phosphat, -succinat, -linoleat, -oleat, Tocophereth-5, Tocophereth-10, Tocophereth-12, Tocophereth-18, Tocophereth-50 und Tocophersolan. Tocopherol und seine Derivate sind bevorzugt in Mengen von 0,05 - 1 Gew.-%, bezogen auf die gesamte Zusammensetzung, enthalten.
Unter Vitamin F werden üblicherweise essentielle Fettsäuren, insbesondere Linolsäure, Linolensäure und Arachidonsäure, verstanden.
Vitamin H ist eine andere Bezeichnung für Biotin oder Vitamin B₇ (siehe oben).
Zu den fettlöslichen Vitaminen der Vitamin K-Gruppe, denen das Grundgerüst des 2-Methyl-1,4-naphthochinons zugrunde liegt, gehören Phyllochinon (Vitamin K₁), Farnochinon oder Menachinon-7 (Vitamin K2) und Menadion (Vitamin K₃). Vitamin K ist bevorzugt in Mengen von 0,0001 bis 1,0 Gew.-%, insbesondere 0,01 bis 0,5 Gew.-%, jeweils bezogen auf die gesamte Zusammensetzung, enthalten.
Vitamin A-palmitat (Retinylpalmitat), Panthenol, Pantolacton, Nicotinsäureamid, Pyridoxin, Pyridoxamin, Pyridoxal, Biotin, Ascorbylpalmitat, -acetat, Mg-Ascorbylphosphat, Na-Ascorbylphosphat, Natrium- und Magnesiumascorbat und die Tocopherolester, besonders Tocopherylacetat, sind erfindungsgemäß besonders bevorzugt.

In einer weiteren bevorzugten Ausführungsform enthalten die erfindungsgemäßen Zusammensetzungen mindestens eine α-Hydroxycarbonsäure, α-Ketocarbonsäure oder β-Hydroxycarbonsäure oder deren Ester-, Lacton- oder Salzform. Es wurde überraschend gefunden, dass ein Gehalt an α-Hydroxycarbonsäure, α-Ketocarbonsäure oder β-Hydroxycarbonsäure oder deren Ester-, Lacton- oder Salzform den optischen Kaschiereffekt der erfindungsgemäßen Zusammensetzungen in unerwartet hohem Ausmaß steigern kann. Erfindungsgemäß bevorzugte α-Hydroxycarbonsäuren oder α-Ketocarbonsäuren sind ausgewählt aus Glycolsäure, Milchsäure, Weinsäure, Citronensäure, 2-Hydroxybutansäure, 2,3-Dihydroxypropansäure, 2-Hydroxypentansäure, 2-Hydroxyhexansäure, 2-Hydroxyheptansäure, 2-Hydroxyoctansäure, 2-Hydroxydecansäure, 2-Hydroxydodecansäure, 2-Hydroxytetradecansäure, 2-Hydroxyhexadecansäure, 2-Hydroxyoctadecansäure, Mandelsäure, 4-Hydroxymandelsäure, Äpfelsäure, Erythrarsäure, Threarsäure, Glucarsäure, Galactarsäure, Mannarsäure, Gularsäure, 2-Hydroxy-2-methylbernsteinsäure, Gluconsäure, Brenztraubensäure, Glucuronsäure und Galacturonsäure. Besonders bevorzugte α-Hydroxycarbonsäuren sind Milchsäure, Citronensäure, Glycolsäure und Gluconsäure. Eine besonders bevorzugte β-Hydroxycarbonsäure ist Salicylsäure. Die Ester der genannten Säuren sind ausgewählt aus den Methyl-, Ethyl-, Propyl-, Isopropyl-, Butyl-, Amyl-, Pentyl-, Hexyl-, 2-Ethylhexyl-, Octyl-, Decyl-, Dodecyl- und Hexadecylestern. Die α-Hydroxycarbonsäuren, α-Ketocarbonsäuren oder β-Hydroxycarbonsäuren oder ihre Derivate sind in den erfindungsgemäßen Zusammensetzungen bevorzugt in einer Gesamtmenge von 0,1 - 10 Gew.-%, besonders bevorzugt 0,5 - 5 Gew.-%, jeweils bezogen auf die gesamte Zusammensetzung, enthalten.

In einer weiteren bevorzugten Ausführungsform enthalten die erfindungsgemäßen Zusammensetzungen mindestens ein Flavonoid oder mindestens einen Flavonoid-reichen Pflanzenextrakt. Auch hier konnte überraschend gefunden werden, dass ein Gehalt an Flavonoid(en) den optischen Kaschiereffekt der erfindungsgemäßen Zusammensetzungen in unerwartet hohem Ausmaß steigern kann.
Die erfindungsgemäß bevorzugten Flavonoide umfassen die Glycoside der Flavone, der Flavanone, der 3-Hydroxyflavone (Flavonole), der Aurone und der Isoflavone. Besonders bevorzugte Flavonoide sind ausgewählt aus Naringin (Aurantiin, Naringenin-7-rhamnoglucosid), α-Glucosylrutin, α-Glucosylmyricetin, α-Glucosylisoquercetin, α-Glucosylquercetin, Hesperidin (3',5,7-Trihydroxy-4'-methoxyflavanon-7-rhamnoglucosid, Hesperitin-7-O-rhamnoglucosid), Neohesperidin, Rutin (3,3',4',5,7-Pentahydroxyflavon-3-rhamnoglucosid, Quercetin-3-rhamnoglucosid), Troxerutin (3,5-Dihydroxy-3',4',7-tris(2-hydroxy-ethoxy)-flavon-3-(6-O-(6-deoxy-α-L-mannopyranosyl)-β-D-glucopyranosid)), Monoxerutin (3,3',4',5-Tetrahydroxy-7-(2-hydroxyethoxy)-flavon-3-(6-O-(6-deoxy-α-L-mannopyranosyl)-β-D-glucopyranosid)), Diosmin (3',4',7-Trihydroxy-5-methoxyflavanon-7-rhamnoglucosid), Eriodictin und Apigenin-7-glucosid (4',5,7-Trihydroxyflavon-7-glucosid).
Erfindungsgemäß außerordentlich bevorzugte Flavonoide sind α-Glucosylrutin, Naringin und Apigenin-7-glucosid.
Ebenfalls bevorzugt sind die aus zwei Flavonoideinheiten aufgebauten Biflavonoide, die z. B. in Gingko-Arten vorkommen. Weitere bevorzugte Flavonoide sind die Chalkone, vor allem Phloricin und Neohesperidindihydrochalkon.
Erfindungsgemäß werden die Flavonoide bevorzugt in Mengen von 0,0001 bis 1 Gew.-%, besonders bevorzugt 0,0005 bis 0,5 Gew.-% und außerordentlich bevorzugt 0,001 bis 0,1 Gew.-%, jeweils bezogen auf die Flavonoidaktivsubstanz in der gesamten W/O-Emulsion, eingesetzt.
In einer weiteren bevorzugten Ausführungsform enthalten die erfindungsgemäßen Zusammensetzungen mindestens ein Isoflavonoid oder mindestens einen Isoflavonoid-reichen Pflanzenextrakt. Zu den Isoflavonoiden werden an dieser Stelle die Isoflavone und die Isoflavon-Glycoside gezählt. Auch hier konnte überraschend gefunden werden, dass ein Gehalt an Isoflavonoid(en) den optischen Kaschiereffekt der erfindungsgemässen Zusammensetzungen in unerwartet hohem Ausmaß steigern kann.
Unter Isoflavonen sind im Sinne der vorliegenden Erfindung Stoffe zu verstehen, die Hydrierungs-, Oxidations- oder Substitutionsprodukte des 3-Phenyl-4H-1-benzopyrans darstellen, wobei eine Hydrierung in der 2,3-Stellung des Kohlenstoffgerüsts vorliegen kann, eine Oxidation unter Ausbildung einer Carbonylgruppe in der 4-Stellung vorliegen kann, und unter Substitution der Ersatz eines oder mehrerer Wasserstoffatome durch Hydroxy- oder Methoxy-Gruppen zu verstehen ist. Zu den erfindungsgemäß bevorzugten Isoflavonen zählen beispielsweise Daidzein, Genistein, Prunetin, Biochanin, Orobol, Santal, Pratensein, Irigenin, Glycitein, Biochanin A und Formononetin. Als Isoflavone besonders bevorzugt sind Daidzein, Genistein, Glycitein und Formononetin.
In den erfindungsgemäß bevorzugten Isoflavon-Glycosiden sind die Isoflavone über mindestens eine Hydroxygruppe mit mindestens einem Zucker glycosidisch verknüpft. Als Zucker kommen Mono- oder Oligosaccharide, insbesondere D-Glucose, D-Galactose, D-Glucuronsäure, D-Galacturonsäure, D-Xylose, D-Apiose, L-Rhamnose, L-Arabinose und Rutinose in Betracht. Erfindungsgemäß besonders bevorzugte Isoflavon-Glycoside sind Daidzin und Genistin.
Erfindungsgemäß weiterhin bevorzugt ist es, wenn die Isoflavone und/oder deren Glycoside als Bestandteile eines aus einer Pflanze gewonnenen Substanzgemisches, insbesondere eines pflanzlichen Extraktes, in den Zubereitungen enthalten sind. Solche pflanzlichen Substanzgemische können in dem Fachmann geläufiger Weise beispielsweise durch Auspressen oder Extrahieren aus Pflanzen wie Soja, Rotklee oder Kichererbsen gewonnen werden. Besonders bevorzugt werden in den erfindungsgemäßen Zubereitungen Isoflavone oder Isoflavon-Glycoside in Form von aus Soja gewonnenen Extrakten eingesetzt, wie sie beispielsweise unter der Produktbezeichnung Soy Protein Isolate SPI (Protein Technology International, St. Louis) oder Soy Phytochemicals Concentrate SPC (Archer Daniels Midland, Decatur) im Handel erhältlich sind. Ein weiterer besonders bevorzugter Isoflavonoid-reicher Pflanzenextrakt ist Apfelkernextrakt, insbesondere das Handelsprodukt Ederline von Seporga. Ederline enthält Phytohormone, Isoflavonoide, Phytosterole, Triterpenoide, Tocopherole und natürliche Wachse.
Erfindungsgemäß werden die lsoflavonoide bevorzugt in Mengen von 0,00001 bis 1 Gew.-%, besonders bevorzugt 0,0005 bis 0,5 Gew.-% und außerordentlich bevorzugt 0,001 bis 0,1 Gew.-%, jeweils bezogen auf die Isoflavonoidaktivsubstanz in der gesamten W/O-Emulsion, eingesetzt.

In einer weiteren bevorzugten Ausführungsform enthalten die erfindungsgemäßen W/O-Emulsionen mindestens ein Polyphenol oder einen Polyphenol-reichen Pflanzenextrakt. Unter Polyphenolen sind erfindungsgemäß aromatische Verbindungen zu verstehen, die mindestens zwei phenolische Hydroxy-Gruppen im Molekül enthalten. Hierzu zählen die drei Dihydroxybenzole Brenzcatechin, Resorcin und Hydrochinon, weiterhin Phloroglucin, Pyrogallol und Hexahydroxybenzol. In der Natur treten freie und veretherte Polyphenole beispielsweise in Blütenfarbstoffen (Anthocyanidine, Flavone), in Gerbstoffen (Catechine, Tannine), als Flechten- oder Farn-Inhaltsstoffe (Usninsäure, Acylpolyphenole), in Ligninen und als Gallussäure-Derivate auf. Bevorzugte Polyphenole sind Flavone, Catechine, Usninsäure, und als Tannine die Derivate der Gallussäure, Digallussäure und Digalloylgallussäure. Besonders bevorzugte Polyphenole sind die monomeren Catechine, das heißt die Derivate der Flavan-3-ole, und Leukoanthocyanidine, das heißt die Derivate der Leukoanthocyanidine, die bevorzugt in 5,7,3',4',5'-Stellung phenolische Hydroxygruppen tragen, bevorzugt Epicatechin und Epigallocatechin, sowie die daraus durch Selbstkondensation entstehenden Gerbstoffe. Solche Gerbstoffe werden bevorzugt nicht in isolierter Reinsubstanz, sondern als Extrakte gerbstoffreicher Pflanzenteile eingesetzt, z. B. Extrakte von Catechu, Quebracho, Eichenrinde und Pinienrinde sowie anderen Baumrinden, Blättern von Grünem Tee (camellia sinensis) und Mate. Ebenfalls besonders bevorzugt sind die Tannine.
Ein besonders bevorzugter Polyphenol-reicher kosmetischer Wirkstoff ist das Handelsprodukt Sepivinol R, ein Extrakt aus Rotwein, erhältlich von der Firma Seppic. Ein weiterer besonders bevorzugter Polyphenol-reicher kosmetischer Wirkstoff ist das Handelsprodukt Crodarom Chardonnay, ein Extrakt aus den Kernen der Chardonnay-Traube, erhältlich von der Firma Croda.
Erfindungsgemäß werden die Polyphenole bevorzugt in Mengen von 0,001 bis 10 Gew.-%, besonders bevorzugt 0,005 bis 5 Gew.-% und außerordentlich bevorzugt 0,01 bis 3 Gew.-%, jeweils bezogen auf die gesamte W/O-Emulsion, eingesetzt.

Weiterhin konnte überraschend beobachtet werden, dass ein Gehalt an Ubichinon(en) oder Ubichinol(en) den optischen Kaschiereffekt der erfindungsgemäßen Zusammensetzungen in unerwartet hohem Ausmaß steigern kann. In einer weiteren bevorzugten Ausführungsform enthalten die erfindungsgemäßen Zusammensetzungen mindestens ein Ubichinon oder ein Ubichinol oder deren Derivate. Ubichinole sind die reduzierte Form der Ubichinone. Die erfindungsgemäß bevorzugten Ubichinone weisen die Formel (II) auf: 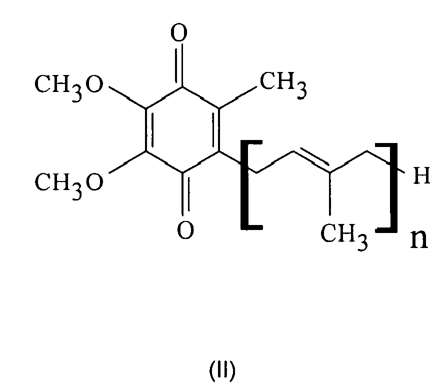 mit n = 6, 7, 8, 9 oder 10.
Besonders bevorzugt ist das Ubichinon der Formel (II) mit n = 10, auch bekannt als Coenzym Q10.
Erfindungsgemäß werden die Ubichinone, Ubichinole oder deren Derivate bevorzugt in Mengen von 0,0001 bis 1 Gew.-%, besonders bevorzugt 0,001 bis 0,5 Gew.-% und außerordentlich bevorzugt 0,005 bis 0,1 Gew.-%, jeweils bezogen auf die gesamte Zusammensetzung, eingesetzt.

In einer weiteren bevorzugten Ausführungsform enthalten die erfindungsgemäßen Zusammensetzungen Silymarin. Es konnte überraschend beobachtet werden, dass ein Gehalt an Silymarin den optischen Kaschiereffekt der erfindungsgemäßen Zusammensetzungen in unerwartet hohem Ausmaß steigern kann. Silymarin stellt erfindungsgemäß ein früher als einheitliche Substanz angesehenes Wirkstoff-Konzentrat aus den Früchten der Mariendistel (Silybum marianum) dar. Die Hauptbestandteile des Silymarins sind Silybin (Silymarin I), Silychristin (Silymarin II) und Silydianin, die zur Gruppe der Flavanolignane gehören.
Erfindungsgemäß wird Silymarin bevorzugt in Mengen von 0,0001 bis 1 Gew.-%, besonders bevorzugt 0,001 bis 0,5 Gew.-% und außerordentlich bevorzugt 0,005 bis 0,1 Gew.-%, jeweils bezogen auf die gesamte Zusammensetzung, eingesetzt.
In einer weiteren bevorzugten Ausführungsform enthalten die erfindungsgemäßen Zusammensetzungen mindestens ein natürlich vorkommendes Xanthin-Derivat, ausgewählt aus Coffein, Theophyllin, Theobromin und Aminophyllin.
Erfindungsgemäß sind die natürlich vorkommenden Xanthin-Derivate bevorzugt in Mengen von 0,0001 bis 1 Gew.-%, besonders bevorzugt 0,001 bis 0,5 Gew.-% und außerordentlich 0,005 bis 0,1 Gew.-%, jeweils bezogen auf die gesamte Zusammensetzung, enthalten.

In einer weiteren bevorzugten Ausführungsform enthalten die erfindungsgemäßen Zusammensetzungen Ectoin. Ectoin ist der Trivialname für 2-Methyl-1,4,5,6-tetrahydropyrimidin-4-carboxylat. Erfindungsgemäß ist Ectoin bevorzugt in Mengen von 0,0001 bis 1 Gew.-%, besonders bevorzugt 0,001 bis 0,5 Gew.-% und außerordentlich bevorzugt 0,005 bis 0,01 Gew.-%, jeweils bezogen auf die gesamte Zusammensetzung, enthalten.

In einer weiteren bevorzugten Ausführungsform enthalten die erfindungsgemäßen Zusammensetzungen mindestens eine anorganische und/oder mindestens eine organische UV-Filtersubstanz.
Bei den UV-Filtersubstanzen handelt es sich um bei Raumtemperatur flüssig oder kristallin vorliegende Substanzen, die in der Lage sind, ultraviolette Strahlen zu absorbieren und die aufgenommene Energie in Form längerwelliger Strahlung, z. B. Wärme, wieder abzugeben. Man unterscheidet UVA-Filter und UVB-Filter. Die UVA- und UVB-Filter können sowohl einzeln als auch in Mischungen eingesetzt werden. Der Einsatz von Filter-Mischungen ist erfindungsgemäß bevorzugt.
Die erfindungsgemäß verwendeten organischen UV-Filter sind ausgewählt aus den physiologisch verträglichen Derivaten von Dibenzoylmethan, Zimtsäureestern, Diphenylacrylsäureestern, Benzophenon, Campher, p-Aminobenzoesäureestern, o-Aminobenzoesäureestern, Salicylsäureestern, Benzimidazolen, symmetrisch oder unsymmetrisch substituierten 1,3,5-Triazinen, monomeren und oligomeren 4,4-Diarylbutadiencarbonsäureestern und -carbonsäureamiden, Ketotricyclo(5.2.1.0)decan, Benzalmalonsäureestern, Benzoxazol sowie beliebigen Mischungen der genannten Komponenten. Die organischen UV-Filter können öllöslich oder wasserlöslich sein. Erfindungsgemäß besonders bevorzugte öllösliche UV-Filter sind 1-(4-tert.-Butylphenyl)-3-(4'-methoxyphenyl)propan-1,3-dion (Parsol® 1789), 1-Phenyl-3-(4'-isopropylphenyl)-propan-1,3-dion, 3-(4'-Methylbenzyliden)-D,L-campher, 4-(Dimethylamino)-benzoesäure-2-ethylhexylester, 4-(Dimethylamino)benzoesäure-2-octylester, 4-(Dimethylamino)-benzoesäureamylester, 4-Methoxyzimtsäure-2-ethylhexylester, 4-Methoxyzimtsäurepropylester, 4-Methoxyzimtsäureisopentylester, 2-Cyano-3,3-phenylzimtsäure-2-ethylhexylester (Octocrylene), Salicylsäure-2-ethylhexylester, Salicylsäure-4-isopropylbenzylester, Salicylsäurehomomenthylester (3,3,5-Trimethyl-cyclohexylsalicylat), 2-Hydroxy-4-methoxybenzophenon, 2-Hydroxy-4-methoxy-4'-methylbenzophenon, 2,2'-Dihydroxy-4-methoxybenzophenon, 4-Methoxybenzmalonsäuredi-2-ethylhexylester, 2,4,6-Trianilino-(p-carbo-2'-ethyl-1'-hexyloxy)-1,3,5-triazin (Octyl Triazone, Uvinul® T 150), Dimethicodiethylbenzal malonate (CAS-Nr. 207574-74-1, Parsol® SLX), Dioctyl Butamido Triazone (Uvasorb® HEB), 2,4-bis-[5-1(di- methylpropyl)benzoxazol-2-yl-(4-phenyl)-imino]-6-(2-ethylhexyl)-imino-1,3,5-triazin (CAS Nr. 288254-16-0, Uvasorb® K2A) und sowie beliebige Mischungen der genannten Komponenten.
Bevorzugte wasserlösliche UV-Filter sind 2-Phenylbenzimidazol-5-sulfonsäure, Phenylen-1,4-bis-(2-benzimidazyl)-3,3'-5,5'-tetrasulfonsäure und deren Alkali-, Erdalkali-, Ammonium-, Alkylammonium-, Alkanolammonium- und Glucammoniumsalze, Sulfonsäurederivate von Benzophenonen, vorzugsweise 2-Hydroxy-4-methoxybenzophenon-5-sulfonsäure und ihre Salze, Sulfonsäurederivate des 3-Benzylidencamphers, wie z. B. 4-(2-Oxo-3-bornylidenmethyl)benzolsulfonsäure und 2-Methyl-5-(2-oxo-3-bornyliden)sulfonsäure und deren Salze.
Einige der öllöslichen UV-Filter können selbst als Lösungsmittel oder Lösungsvermittler für andere UV-Filter dienen. So lassen sich beispielsweise Lösungen des UV-A-Filters 1-(4-tert.-Butylphenyl)-3-(4'methoxyphenyl)propan-1,3-dion (z. B. Parsol® 1789) in verschiedenen UV-B-Filtern herstellen. Die erfindungsgemäßen Zusammensetzungen enthalten daher in einer weiteren bevorzugten Ausführungsform 1-(4-tert.-Butylphenyl)-3-(4'-methoxyphenyl)propan-1,3-dion in Kombination mit mindestens einem UV-B-Filter, ausgewählt aus 4-Methoxyzimtsäure-2-ethylhexylester, 2-Cyano-3,3-phenylzimtsäure-2-ethylhexylester, Salicylsäure-2-ethylhexylester und 3,3,5-Trimethyl-cyclohexylsalicylat. In diesen Kombinationen liegt das Gewichtsverhältnis von UV-B-Filter zu 1-(4-tert.-Butylphenyl)-3-(4'methoxyphenyl)propan-1,3-dion zwischen 1:1 und 10:1, bevorzugt zwischen 2:1 und 8:1, das molare Verhältnis liegt entsprechend zwischen 0,3 und 3,8, bevorzugt zwischen 0,7 und 3,0, besonders bevorzugt bei etwa 2,5.

Bei den erfindungsgemäß bevorzugten anorganischen Lichtschutzpigmenten handelt es sich um feindisperse oder kolloiddisperse Metalloxide und Metallsalze, beispielsweise Titandioxid, Zinkoxid, Eisenoxid, Aluminiumoxid, Ceroxid, Zirkoniumoxid, Silicate (Talk) und Bariumsulfat. Die Partikel sollten dabei einen mittleren Durchmesser von weniger als 100 nm, vorzugsweise zwischen 5 und 50 nm und insbesondere zwischen 15 und 30 nm aufweisen, so genannte Nanopigmente. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z. B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. Besonders bevorzugt sind Titandioxid und Zinkoxid.
Erfindungsgemäß sind die organischen UV-Filtersubstanzen bevorzugt in Mengen von 0,1 - 30 Gew.-%, besonders bevorzugt 0,5 - 20 Gew.-%, ganz besonders bevorzugt 1,0 - 15 Gew.-% und außerordentlich bevorzugt 3,0 - 10 Gew.-%, jeweils bezogen auf die gesamte Zusammensetzung, enthalten.
Erfindungsgemäß sind die anorganischen UV-Filtersubstanzen bevorzugt in Mengen von 0,1 - 15 Gew.-%, besonders bevorzugt 0,5 - 10 Gew.-%, ganz besonders bevorzugt 1,0 - 5 Gew.-% und außerordentlich bevorzugt 2,0 - 4,0 Gew.-%, jeweils bezogen auf die gesamte Zusammensetzung, enthalten.

In einer weiteren bevorzugten Ausführungsform enthalten die erfindungsgemäßen Zusammensetzungen mindestens einen selbstbräunenden Wirkstoff. Erfindungsgemäß bevorzugte selbstbräunende Wirkstoffe sind ausgewählt aus Dihydroxyaceton und Erythrulose.
Erfindungsgemäß sind die selbstbräunenden Wirkstoffe bevorzugt in Mengen von 0,1 - 15 Gew.-%, besonders bevorzugt 0,5 - 10 Gew.-%, ganz besonders bevorzugt 1,0 - 5 Gew.-% und außerordentlich bevorzugt 2,0 - 4,0 Gew.-%, jeweils bezogen auf die gesamte W/O-Emulsion, enthalten.

In einer weiteren bevorzugten Ausführungsform enthalten die erfindungsgemäßen Zusammensetzungen mindestens einen hautaufhellenden Wirkstoff. Erfindungsgemäß bevorzugte hautaufhellende Wirkstoffe sind ausgewählt aus 9-Z-Octadecendicarbonsäure (erhältlich unter der Bezeichnung Arlatone DIOIC DCA von Uniqema), Ascorbinsäure, den Estern der Ascorbinsäure mit Phosphorsäure und/oder organischen C₂-C₂₀₋Carbonsäuren sowie deren Alkali- und Erdalkalimetallsalzen, Kojisäure, Hydrochinon, Arbutin, Maulbeerbaumextrakt und Süßholzextrakt sowie Mischungen hiervon. Sowohl als Einzelsubstanz wie auch in Mischung sind 9-Z-Octadecendicarbonsäure, die Ascorbinsäurederivate sowie Kojisäure bevorzugt. Besonders bevorzugt sind 9-Z-Octadecendicarbonsäure, Natriumascorbylphosphat, Magnesiumascorbylphosphat, Ascorbylmonopalmitat, Ascorbyldipalmitat, Ascorbylmonostearat, Ascorbyldistearat, Ascorbylmonoethylhexanoat, Ascorbyldiethylhexanoat, Ascorbylmonooctanoat, Ascorbyldioctanoat, Ascorbylmonoisostearat und Ascorbyldiisostearat. Die erfindungsgemäß außerordentlich bevorzugten Ascorbinsäurederivate sind Natriumascorbylphosphat und Magnesiumascorbylphosphat.
Die hautaufhellenden Wirkstoffe sind bevorzugt in einer Menge von 0,05 bis 5 Gew.-%, besonders bevorzugt von 0,1 - 2 Gew-%, jeweils bezogen auf die gesamte Zusammensetzung, enthalten.

In einer weiteren bevorzugten Ausführungsform enthalten die erfindungsgemäßen Zusammensetzungen mindestens einen hautberuhigenden Wirkstoff. Erfindungsgemäß bevorzugte hautberuhigende Wirkstoffe sind ausgewählt aus Allantoin, α-Bisabolol und α-Liponsäure. Die hautberuhigenden Wirkstoffe sind bevorzugt in Mengen von 0,001 bis 5 Gew.-%, besonders bevorzugt 0,01 bis 2 Gew.-% und außerordentlich bevorzugt 0,1 bis 1 Gew.-%, jeweils bezogen auf die gesamte Zusammensetzung, enthalten.

In einer weiteren bevorzugten Ausführungsform enthalten die erfindungsgemäßen Zusammensetzungen mindestens einen Desoxyzucker oder ein mindestens einen Desoxyzucker-Baustein enthaltendes Polysaccharid. Es konnte überraschend beobachtet werden, dass ein Gehalt an Desoxyzucker oder mindestens einem Desoxyzucker-Bausteine enthaltenden Polysaccharid den optischen Kaschiereffekt der erfindungsgemäßen Zusammensetzungen in unerwartet hohem Ausmaß steigern kann.
Erfindungsgemäß bevorzugte Desoxyzucker sind ausgewählt aus Rhamnose und Fucose. Erfindungsgemäß besonders bevorzugte, Desoxyzucker-Bausteine enthaltende Polysaccharid sind die Handelsprodukte Fucogel® (INCI-Bezeichnung Biosaccharide Gum-1) von Solabia, Rhamnosoft® (INCI-Bezeichnung Biosaccharide Gum-2) von Solabia, Fucogenol® (INCI-Bezeichnung Biosaccharide Gum-3) von Solabia und Glycofilm® (INCI-Bezeichnung Biosaccharide Gum-4) von Solabia, weiterhin Mischungen der vorgenannten, mindestens einen Desoxyzucker-Baustein enthaltenden Polysaccharide, beispielsweise eine Mischung aus Biosaccharide Gum-2 und Biosaccharide Gum-3, erhältlich als Handelsprodukt Elastinol plus® von Solabia, sowie beliebige Mischungen dieser Substanzen.

Die Desoxyzucker oder Desoxyzucker-Bausteine enthaltenden Polysaccharide sind bevorzugt in Mengen von 0,001 bis 5 Gew.-%, besonders bevorzugt 0,01 bis 2 Gew.-% und außerordentlich bevorzugt 0,1 bis 1 Gew.%, jeweils bezogen auf die gesamte Zusammensetzung, enthalten.

Ein weiterer Gegenstand der vorliegenden Erfindung ist die Verwendung einer kosmetischen Zusammensetzung in Form einer Creme oder Lotion auf Basis einer Wasser-in-Siliconöl-Emulsion, enthaltend 15-35 Gew.-% einer Fettphase mit einem Anteil an mindestens einem Siliconöl von 70 - 95 Gew.-%, bezogen auf das Gewicht der Fettphase, Cetyl PEG/PPG-10/1 Dimethicone als Wasser-in-Öl-Siliconemulgator, 50 - 90 Gew.-% Wasserphase, bezogen auf die Gesamtzusammensetzung, 0,5 bis weniger als 3 Gew.-% mindestens einer Lipid- oder Wachskomponente, wobei der Schmelzpunkt der Lipid-oder Wachskomponente oberhalb bei 60°C oder darüber liegt oder, falls mehrere Lipid-oder Wachskomponenten enthalten sind, der Schmelzpunkt dieser Mischung von Lipid- oder Wachskomponenten bei 60°C oder darüber liegt, und wobei die Lipid- oder Wachskomponente mit einem Schmelzpunkt von 60°C oder darüber ausgewählt ist aus Estern aus einem gesättigten, einwertigen C₁₆-C₆₀-Alkanol und einer gesättigten C₈-C₃₆-Monocarbonsäure, Glycerintriestern von gesättigten linearen C₁₈ - C₄₀-Carbonsäuren, die hydroxyliert sein können, Fruchtwachsen, Pflanzenwachsen, insbesondere Candelillawachs und Carnaubawachs, Insektenwachsen, insbesondere Bienenwachs, gesättigten linearen C₁₆ -, C₁₈ -, C₂₀ - und C₂₂ - C₄₀-Carbonsäuren, gehärtetem Ricinusöl, Ozokerit, Mikrowachsen, Ceresin, Paraffinwachsen, sowie solchen Mischungen der vorgenannten Substanzen, deren Schmelzpunkt bei 60°C oder darüber liegt, und 0,5 - 7 Gew.-% mindestens eines Pigments mit einer mittleren Partikelgröße von 0,5 - 25 µm, das im Wellenlängenbereich von 400 - 800 nm diffuse Lichtstreuungseigenschaften und einen Transmissionswert von 60 bis mindestens 95 % aufweist und das ausgewählt ist aus sphärischen Kieselsäurepartikeln, die mit Titandioxid (CI 77891) und Eisenoxid (CI 77491) beschichtet sind, sphärischen Partikeln aus vernetztem Polymethylmethacrylat, sphärischen TiO₂-haltige Kieselsäurepartikeln sowie Mischungen der genannten Pigmente, zur nicht-therapeutischen, kosmetischen Behandlung und/oder optischen Kaschierung von feinen Linien, Hautfalten und/ oder Hautfältchen, den Anzeichen der intrinsischen und extrinsischen Hautalterung, müder und/oder schlaffer Haut, trockener Haut, Altersflecken und gutartigen Pigmentstörungen, fettiger und/oder unreiner Haut, UV-geschädigter Haut und/oder gereizter Haut.

Ein weiterer Gegenstand der vorliegenden Erfindung ist ein Verfahren zur nicht-therapeutischen kosmetischen Hautbehandlung, bei der eine kosmetische Zusammensetzung in Form einer Creme oder Lotion auf Basis einer Wasser-in-Siliconöl-Emulsion, enthaltend enthaltend 15 - 35 Gew.-% einer Fettphase mit einem Anteil an mindestens einem Siliconöl von 70 - 95 Gew.-%, bezogen auf das Gewicht der Fettphase, Cetyl PEG/PPG-10/1 Dimethicone als Wasser-in-Öl-Siliconemulgator, 50 - 90 Gew.-% Wasserphase, bezogen auf die Gesamtzusammensetzung, 0,5 bis weniger als 3 Gew.-% mindestens einer Lipid- oder Wachskomponente, wobei der Schmelzpunkt der Lipid- oder Wachskomponente oberhalb bei 60°C oder darüber liegt oder, falls mehrere Lipid- oder Wachskomponenten enthalten sind, der Schmelzpunkt dieser Mischung von Lipid- oder Wachskomponenten bei 60°C oder darüber liegt, und wobei die Lipid- oder Wachskomponente mit einem Schmelzpunkt von 60°C oder darüber ausgewählt ist aus Estern aus einem gesättigten, einwertigen C₁₆-C₆₀-Alkanol und einer gesättigten C₈-C₃₆-Monocarbonsäure, Glycerintriestern von gesättigten linearen C₁₈ - C₄₀-Carbonsäuren, die hydroxyliert sein können, Fruchtwachsen, Pflanzenwachsen, insbesondere Candelillawachs und Carnaubawachs, Insektenwachsen, insbesondere Bienenwachs, gesättigten linearen C₁₆ -, C₁₈ -, C₂₀ - und C₂₂ - C₄₀-Carbonsäuren, gehärtetem Ricinusöl, Ozokerit, Mikrowachsen, Ceresin, Paraffinwachsen, sowie solchen Mischungen der vorgenannten Substanzen, deren Schmelzpunkt bei 60°C oder darüber liegt, und 0,5 - 7 Gew.-% mindestens eines Pigments mit einer mittleren Partikelgröße von 0,5 - 25 µm, das im Wellenlängenbereich von 400 - 800 nm diffuse Lichtstreuungseigenschaften und einen Transmissionswert von 60 bis mindestens 95 % aufweist und das ausgewählt ist aus sphärischen Kieselsäurepartikeln, die mit Titandioxid (CI 77891) und Eisenoxid (CI 77491) beschichtet sind, sphärischen Partikeln aus vernetztem Polymethylmethacrylat, sphärischen TiO₂-haltigen Kieselsäurepartikeln sowie Mischungen der genannten Pigmente, auf die Haut, insbesondere die Gesichtshaut und bevorzugt auf die feinen Linien, Hautfalten und/oder Hautfältchen, die Anzeichen der intrinsischen und extrinsischen Hautalterung, auf müde und/oder schlaffe Haut, trockene Haut, Altersflecken und gutartige Pigmentstörungen, fettige und/oder unreine Haut, UV-geschädigte und/oder gereizte Haut, aufgetragen wird.
Ein weiterer Gegenstand der vorliegenden Erfindung ist ein Verfahren zur nicht-therapeutischen kosmetischen Hautbehandlung, bei der eine kosmetische Zusammensetzung in Form einer Creme oder Lotion auf Basis einer Wasser-in-Siliconöl-Emulsion, enthaltend enthaltend 15-35 Gew.-% einer Fettphase mit einem Anteil an mindestens einem Siliconöl von 70 - 95 Gew.-%, bezogen auf das Gewicht der Fettphase, Cetyl PEG/PPG-10/1 Dimethicone als Wasser-in-Öl-Siliconemulgator, 50 - 90 Gew.-% Wasserphase, bezogen auf die Gesamtzusammensetzung, 0,5 bis weniger als 3 Gew.-% mindestens einer Lipid- oder Wachskomponente, wobei der Schmelzpunkt der Lipid- oder Wachskomponente oberhalb bei 60°C oder darüber liegt oder, falls mehrere Lipid- oder Wachskomponenten enthalten sind, der Schmelzpunkt dieser Mischung von Lipid- oder Wachskomponenten bei 60°C oder darüber liegt, und wobei die Lipid- oder Wachskomponente mit einem Schmelzpunkt von 60°C oder darüber ausgewählt ist aus Estern aus einem gesättigten, einwertigen C₁₆-C₆₀-Alkanol und einer gesättigten C₈-C₃₆-Monocarbonsäure, Glycerintriestern von gesättigten linearen C₁₈ - C₄₀-Carbonsäuren, die hydroxyliert sein können, Fruchtwachsen, Pflanzenwachsen, insbesondere Candelillawachs und Carnaubawachs, Insektenwachsen, insbesondere Bienenwachs, gesättigten linearen C₁₆ -, C₁₈ -, C₂₀ - und C₂₂ - C₄₀-Carbonsäuren, gehärtetem Ricinusöl, Ozokerit, Mikrowachsen, Ceresin, Paraffinwachsen, sowie solchen Mischungen der vorgenannten Substanzen, deren Schmelzpunkt bei 60°C oder darüber liegt, und 0,5 -7 Gew.-% mindestens eines Pigments mit einer mittleren Partikelgröße von 0,5 - 25 µm, das im Wellenlängenbereich von 400 - 800 nm diffuse Lichtstreuungseigenschaften und einen Transmissionswert von 60 bis mindestens 95 % aufweist und das ausgewählt ist aus sphärischen Kieselsäurepartikeln, die mit Titandioxid (CI 77891) und Eisenoxid (CI 77491) beschichtet sind, sphärischen Partikeln aus vernetztem Polymethylmethacrylat, sphärischen TiO₂-haltigen Kieselsäurepartikeln sowie Mischungen der genannten Pigmente, sowie weiterhin mindestens ein Feuchthaltemittel, auf die Haut, insbesondere die Gesichtshaut und bevorzugt auf die feinen Linien, Hautfalten und/oder Hautfältchen, die Anzeichen der intrinsischen und extrinsischen Hautalterung, auf müde und/oder schlaffe Haut, trockene Haut, Altersflecken und gutartige Pigmentstörungen, fettige und/oder unreine Haut, UV-geschädigte und/oder gereizte Haut, aufgetragen wird.
Ein weiterer Gegenstand der vorliegenden Erfindung ist ein Verfahren zur nicht-therapeutischen kosmetischen Hautbehandlung, bei der eine kosmetische Zusammensetzung in Form einer Creme oder Lotion auf Basis einer Wasser-in-Siliconöl-Emulsion, enthaltend enthaltend 15-35 Gew.-% einer Fettphase mit einem Anteil an mindestens einem Siliconöl von 70 - 95 Gew.-%, bezogen auf das Gewicht der Fettphase, Cetyl PEG/PPG-10/1 Dimethicone als Wasser-in-Öl-Siliconemulgator, 50 - 90 Gew.-% Wasserphase, bezogen auf die Gesamtzusammensetzung, 0,5 bis weniger als 3 Gew.-% mindestens einer Lipid- oder Wachskomponente, wobei der Schmelzpunkt der Lipid- oder Wachskomponente oberhalb bei 60°C oder darüber liegt oder, falls mehrere Lipid- oder Wachskomponenten enthalten sind, der Schmelzpunkt dieser Mischung von Lipid- oder Wachskomponenten bei 60°C oder darüber liegt, und wobei die Lipid- oder Wachskomponente mit einem Schmelzpunkt von 60°C oder darüber ausgewählt ist aus Estern aus einem gesättigten, einwertigen C₁₆-C₆₀₋Alkanol und einer gesättigten C₈-C₃₆-Monocarbonsäure, Glycerintriestern von gesättigten linearen C₁₈ - C₄₀-Carbonsäuren, die hydroxyliert sein können, Fruchtwachsen, Pflanzenwachsen, insbesondere Candelillawachs und Carnaubawachs, Insektenwachsen, insbesondere Bienenwachs, gesättigten linearen C₁₆ - C₁₈ -, C₂₀ - und C₂₂ - C₄₀-Carbonsäuren, gehärtetem Ricinusöl, Ozokerit, Mikrowachsen, Ceresin, Paraffinwachsen, sowie solchen Mischungen der vorgenannten Substanzen, deren Schmelzpunkt bei 60°C oder darüber liegt, und 0,5 - 7 Gew.-% mindestens eines Pigments mit einer mittleren Partikelgröße von 0,5 - 25 µm, das im Wellenlängenbereich von 400 - 800 nm diffuse Lichtstreuungseigenschaften und einen Transmissionswert von 60 bis mindestens 95 % aufweist und das ausgewählt ist aus sphärischen Kieselsäurepartikeln, die mit Titandioxid (CI 77891) und Eisenoxid (CI 77491) beschichtet sind, sphärischen Partikeln aus vernetztem Polymethylmethacrylat, sphärischen TiO₂-haltige Kieselsäurepartikeln sowie Mischungen der genannten Pigmente, sowie weiterhin mindestens ein Monomer, Oligomer oder Polymer von Aminosäuren, N-C₂-C₂₄-Acylaminosäuren, den Estern und/oder den physiologisch verträglichen Metallsalzen dieser Substanzen, auf die Haut, insbesondere die Gesichtshaut und bevorzugt auf die feinen Linien, Hautfalten und/oder Hautfältchen, die Anzeichen der intrinsischen und extrinsischen Hautalterung, auf müde und/oder schlaffe Haut, trockene Haut, Altersflecken und gutartige Pigmentstörungen, fettige und/oder unreine Haut, UV-geschädigte und/oder gereizte Haut, aufgetragen wird.
Ein weiterer Gegenstand der vorliegenden Erfindung ist ein Verfahren zur nicht-therapeutischen kosmetischen Hautbehandlung, bei der eine kosmetische Zusammensetzung in Form einer Creme oder Lotion auf Basis einer Wasser-in-Siliconöl-Emulsion, enthaltend enthaltend 15-35 Gew.-% einer Fettphase mit einem Anteil an mindestens einem Siliconöl von 70 - 95 Gew.%, bezogen auf das Gewicht der Fettphase, Cetyl PEG/PPG-10/1 Dimethicone als Wasser-in-Öl-Siliconemulgator, 50 - 90 Gew.-% Wasserphase, bezogen auf die Gesamtzusammensetzung, 0,5 bis weniger als 3 Gew.-% mindestens einer Lipid- oder Wachskomponente, wobei der Schmelzpunkt der Lipid- oder Wachskomponente oberhalb bei 60°C oder darüber liegt oder, falls mehrere Lipide oder Wachskomponenten enthalten sind, der Schmelzpunkt dieser Mischung von Lipid- oder Wachskomponenten bei 60°C oder darüber liegt, und wobei die Lipid- oder Wachskomponente mit einem Schmelzpunkt von 60°C oder darüber ausgewählt ist aus Estern aus einem gesättigten, einwertigen C₁₆-C₆₀-Alkanol und einer gesättigten C₈-C₃₈-Monocarbonsäure, Glycerintriestern von gesättigten linearen C₁₈ - C₄₀-Carbonsäuren, die hydroxyliert sein können, Fruchtwachsen, Pflanzenwachsen, insbesondere Candelillawachs und Carnaubawachs, Insektenwachsen, insbesondere Bienenwachs, gesättigten linearen C₁₆ -, C₁₈ -, C₂₀ - und C₂₂ - C₄₀-Carbonsäuren, gehärtetem Ricinusöl, Ozokerit, Mikrowachsen, Ceresin, Paraffinwachsen, sowie solchen Mischungen der vorgenannten Substanzen, deren Schmelzpunkt bei 60°C oder darüber liegt, und 0,5 - 7 Gew.-% mindestens eines Pigments mit einer mittleren Partikelgröße von 0,5 - 25 µm, das im Wellenlängenbereich von 400 - 800 nm diffuse Lichtstreuungseigenschaften und einen Transmissionswert von 60 bis mindestens 95 % aufweist und das ausgewählt ist aus sphärischen Kieselsäurepartikeln, die mit Titandioxid (CI 77891) und Eisenoxid (CI 77491) beschichtet sind, sphärischen Partikeln aus vernetztem Polymethylmethacrylat, sphärischen TiO₂-haitigen Kieselsäurepartikeln sowie Mischungen der genannten Pigmente, sowie weiterhin mindestens eine natürliche Betainverbindung, auf die Haut, insbesondere die Gesichtshaut und bevorzugt auf die feinen Linien, Hautfalten und/oder Hautfältchen, die Anzeichen der intrinsischen und extrinsischen Hautalterung, auf müde und/oder schlaffe Haut, trockene Haut, Altersflecken und gutartige Pigmentstörungen, fettige und/oder unreine Haut, UV-geschädigte und/oder gereizte Haut, aufgetragen wird. Ein weiterer Gegenstand der vorliegenden Erfindung ist ein Verfahren zur nicht-therapeutischen kosmetischen Hautbehandlung, bei der eine kosmetische Zusammensetzung in Form einer Creme oder Lotion auf Basis einer Wasser-in-Siliconöl-Emulsion, enthaltend enthaltend 15-35 Gew.-% einer Fettphase mit einem Anteil an mindestens einem Siliconöl von 70 - 95 Gew.-%, bezogen auf das Gewicht der Fettphase, Cetyl PEG/PPG-10/1 Dimethicone als Wasser-in-Öl-Siliconemulgator, 50 - 90 Gew.-% Wasserphase, bezogen auf die Gesamtzusammensetzung, 0,5 bis weniger als 3 Gew.-% mindestens einer Lipid- oder Wachskomponente, wobei der Schmelzpunkt der Lipid- oder Wachskomponente oberhalb bei 60°C oder darüber liegt oder, falls mehrere Lipid- oder Wachskomponenten enthalten sind, der Schmelzpunkt dieser Mischung von Lipid- oder Wachskomponenten bei 60°C oder darüber liegt, und wobei die Lipid- oder Wachskomponente mit einem Schmelzpunkt von 60°C oder darüber ausgewählt ist aus Estern aus einem gesättigten, einwertigen C₁₆-C₆₀-Alkanol und einer gesättigten C₈-C₃₆-Monocarbonsäure, Glycerintriestern von gesättigten linearen C₁₈ - C₄₀-Carbonsäuren, die hydroxyliert sein können, Fruchtwachsen, Pflanzenwachsen, insbesondere Candelillawachs und Carnaubawachs, Insektenwachsen, insbesondere Bienenwachs, gesättigten linearen C₁₆ -, C₁₈ -, C₂₀ - und C₂₂ - C₄₀-Carbonsäuren, gehärtetem Ricinusöl, Ozokerit, Mikrowachsen, Ceresin, Paraffinwachsen, sowie solchen Mischungen der vorgenannten Substanzen, deren Schmelzpunkt bei 60°C oder darüber liegt, und 0,5 - 7 Gew.-% mindestens eines Pigments mit einer mittleren Partikelgröße von 0,5 - 25 µm, das im Wellenlängenbereich von 400 - 800 nm diffuse Lichtstreuungseigenschaften und einen Transmissionswert von 60 bis mindestens 95 % aufweist und das ausgewählt ist aus sphärischen Kieselsäurepartikeln, die mit Titandioxid (CI 77891) und Eisenoxid (CI 77491) beschichtet sind, sphärischen Partikeln aus vernetztem Polymethylmethacrylat, sphärischen TiO₂-haltigen Kieselsäurepartikeln sowie Mischungen der genannten Pigmente, sowie weiterhin mindestens ein Vitamin, Provitamin oder eine Vitaminvorstufe der Gruppen A, B, C, E, H und K oder ein Ester der vorgenannten Substanzen, auf die Haut, insbesondere die Gesichtshaut und bevorzugt auf die feinen Linien, Hautfalten und/oder Hautfältchen, die Anzeichen der intrinsischen und extrinsischen Hautalterung, auf müde und/oder schlaffe Haut, trockene Haut, Altersflecken und gutartige Pigmentstörungen, fettige und/oder unreine Haut, UV-geschädigte und/oder gereizte Haut, aufgetragen wird.
Ein weiterer Gegenstand der vorliegenden Erfindung ist ein Verfahren zur nicht-therapeutischen kosmetischen Hautbehandlung, bei der eine kosmetische Zusammensetzung in Form einer Creme oder Lotion auf Basis einer Wasser-in-Siliconöl-Emulsion, enthaltend enthaltend 15-35 Gew.-% einer Fettphase mit einem Anteil an mindestens einem Siliconöl von 70 - 95 Gew.%, bezogen auf das Gewicht der Fettphase, Cetyl PEG/PPG-10/1 Dimethicone als Wasser-in-Öl-Siliconemulgator, 50 - 90 Gew.-% Wasserphase, bezogen auf die Gesamtzusammensetzung, 0,5 bis weniger als 3 Gew.-% mindestens einer Lipid- oder Wachskomponente, wobei der Schmelzpunkt der Lipid- oder Wachskomponente oberhalb bei 60°C oder darüber liegt oder, falls mehrere Lipid- oder Wachskomponenten enthalten sind, der Schmelzpunkt dieser Mischung von Lipid- oder Wachskomponenten bei 60°C oder darüber liegt, und wobei die Lipid- oder Wachskomponente mit einem Schmelzpunkt von 60°C oder darüber ausgewählt ist aus Estern aus einem gesättigten, einwertigen C₁₆-C₆₀-Alkanol und einer gesättigten C₈-C₃₆-Monocarbonsäure, Glycerintriestern von gesättigten linearen C₁₈ - C₄₀-Carbonsäuren, die hydroxyliert sein können, Fruchtwachsen, Pflanzenwachsen, insbesondere Candelillawachs und Carnaubawachs, Insektenwachsen, insbesondere Bienenwachs, gesättigten linearen C₁₆ -, C₁₈ -, C₂₀ - und C₂₂ - C₄₀-Carbonsäuren, gehärtetem Ricinusöl, Ozokerit, Mikrowachsen, Ceresin, Paraffinwachsen, sowie solchen Mischungen der vorgenannten Substanzen, deren Schmelzpunkt bei 60°C oder darüber liegt, und 0,5 - 7 Gew.-% mindestens eines Pigments mit einer mittleren Partikelgröße von 0,5 - 25 µm, das im Wellenlängenbereich von 400 - 800 nm diffuse Lichtstreuungseigenschaften und einen Transmissionswert von 60 bis mindestens 95 % aufweist und das ausgewählt ist aus sphärischen Kieselsäurepartikeln, die mit Titandioxid (CI 77891) und Eisenoxid (CI 77491) beschichtet sind, sphärischen Partikeln aus vernetztem Polymethylmethacrylat, sphärischen TiO₂-haltigen Kieselsäurepartikeln sowie Mischungen der genannten Pigmente, sowie weiterhin mindestens eine α-Hydroxycarbonsäure, α-Ketocarbonsäure, β-Hydroxycarbonsäure oder deren Ester-, Lacton- oder Salzform, auf die Haut, insbesondere die Gesichtshaut und bevorzugt auf die feinen Linien, Hautfalten und/oder Hautfältchen, die Anzeichen der intrinsischen und extrinsischen Hautalterung, auf müde und/oder schlaffe Haut, trockene Haut, Altersflecken und gutartige Pigmentstörungen, fettige und/oder unreine Haut, UV-geschädigte und/oder gereizte Haut, aufgetragen wird. Ein weiterer Gegenstand der vorliegenden Erfindung ist ein Verfahren zur nicht-therapeutischen kosmetischen Hautbehandlung, bei der eine kosmetische Zusammensetzung in Form einer Creme oder Lotion auf Basis einer Wasser-in-Siliconöl-Emulsion, enthaltend enthaltend 15-35 Gew.-% einer Fettphase mit einem Anteil an mindestens einem Siliconöl von 70 - 95 Gew.-%, bezogen auf das Gewicht der Fettphase, Cetyl PEG/PPG-10/1 Dimethicone als Wasser-in-Öl-Siliconemulgator, 50 - 90 Gew.-% Wasserphase, bezogen auf die Gesamtzusammensetzung, 0,5 bis weniger als 3 Gew.-% mindestens einer Lipid- oder Wachskomponente, wobei der Schmelzpunkt der Lipid- oder Wachskomponente oberhalb bei 60°C oder darüber liegt oder, falls mehrere Lipid- oder Wachskomponenten enthalten sind, der Schmelzpunkt dieser Mischung von Lipid- oder Wachskomponenten bei 60°C oder darüber liegt, und wobei die Lipid- oder Wachskomponente mit einem Schmelzpunkt von 60°C oder darüber ausgewählt ist aus Estern aus einem gesättigten, einwertigen C₁₆-C₆₀-Alkanol und einer gesättigten C₈-C₃₆-Monocarbonsäure, Glycerintriestern von gesättigten linearen C₁₈ - C₄₀-Carbonsäuren, die hydroxyliert sein können, Fruchtwachsen, Pflanzenwachsen, insbesondere Candelillawachs und Carnaubawachs, Insektenwachsen, insbesondere Bienenwachs, gesättigten linearen C₁₆ -, C₁₈ -, C₂₀ - und C₂₂ - C₄₀-Carbonsäuren, gehärtetem Ricinusöl, Ozokerit, Mikrowachsen, Ceresin, Paraffinwachsen, sowie solchen Mischungen der vorgenannten Substanzen, deren Schmelzpunkt bei 60°C oder darüber liegt, und 0,5 - 7 Gew.-% mindestens eines Pigments mit einer mittleren Partikelgröße von 0,5 - 25 µm, das im Wellenlängenbereich von 400 - 800 nm diffuse Lichtstreuungseigenschaften und einen Transmissionswert von 60 bis mindestens 95 % aufweist und das ausgewählt ist aus sphärischen Kieselsäurepartikeln, die mit Titandioxid (CI 77891) und Eisenoxid (CI 77491) beschichtet sind, sphärischen Partikeln aus vernetztem Polymethylmethacrylat, sphärischen TiO₂-haltige Kieselsäurepartikeln sowie Mischungen der genannten Pigmente, sowie weiterhin mindestens ein Ubichinon oder ein Ubichinol oder deren Derivat, auf die Haut, insbesondere die Gesichtshaut und bevorzugt auf die feinen Linien, Hautfalten und/oder Hautfältchen, die Anzeichen der intrinsischen und extrinsischen Hautalterung, auf müde und/oder schlaffe Haut, trockene Haut, Altersflecken und gutartige Pigmentstörungen, fettige und/oder unreine Haut, UV-geschädigte und/oder gereizte Haut, aufgetragen wird.
Ein weiterer Gegenstand der vorliegenden Erfindung ist ein Verfahren zur nicht-therapeutischen kosmetischen Hautbehandlung, bei der eine kosmetische Zusammensetzung in Form einer Creme oder Lotion auf Basis einer Wasser-in-Siliconöl-Emulsion, enthaltend enthaltend 15-35 Gew.-% einer Fettphase mit einem Anteil an mindestens einem Siliconöl von 70 - 95 Gew.%, bezogen auf das Gewicht der Fettphase, Cetyl PEG/PPG-10/1 Dimethicone als Wasser-in-Öl-Siliconemulgator, 50 - 90 Gew.-% Wasserphase, bezogen auf die Gesamtzusammensetzung, 0,5 bis weniger als 3 Gew.-% mindestens einer Lipid- oder Wachskomponente, wobei der Schmelzpunkt der Lipid- oder Wachskomponente oberhalb bei 60°C oder darüber liegt oder, falls mehrere Lipid- oder Wachskomponenten enthalten sind, der Schmelzpunkt dieser Mischung von Lipid- oder Wachskomponenten bei 60°C oder darüber liegt, und wobei die Lipid- oder Wachskomponente mit einem Schmelzpunkt von 60°C oder darüber ausgewählt ist aus Estern aus einem gesättigten, einwertigen C₁₆-C₆₀-Alkanol und einer gesättigten C₈-C₃₆-Monocarbonsäure, Glycerintriestern von gesättigten linearen C₁₈ - C₄₀-Carbonsäuren, die hydroxyliert sein können, Fruchtwachsen, Pflanzenwachsen, insbesondere Candelillawachs und Carnaubawachs, Insektenwachsen, insbesondere Bienenwachs, gesättigten linearen C₁₆ -, C₁₈ -, C₂₀ - und C₂₂ - C₄₀-Carbonsäuren, gehärtetem Ricinusöl, Ozokerit, Mikrowachsen, Ceresin, Paraffinwachsen, sowie solchen Mischungen der vorgenannten Substanzen, deren Schmelzpunkt bei 60°C oder darüber liegt, und 0,5 - 7 Gew.-% mindestens eines Pigments mit einer mittleren Partikelgröße von 0,5 - 25 µm, das im Wellenlängenbereich von 400 - 800 nm diffuse Lichtstreuungseigenschaften und einen Transmissionswert von 60 bis mindestens 95 % aufweist und das ausgewählt ist aus sphärischen Kieselsäurepartikeln, die mit Titandioxid (CI 77891) und Eisenoxid (CI 77491) beschichtet sind, sphärischen Partikeln aus vernetztem Polymethylmethacrylat, sphärischen TiO₂-haltige Kieselsäurepartikeln sowie Mischungen der genannten Pigmente, sowie weiterhin mindestens ein natürlich vorkommendes Xanthin-Derivat, ausgewählt aus Coffein, Theophyllin, Theobromin und Aminophyllin, auf die Haut, insbesondere die Gesichtshaut und bevorzugt auf die feinen Linien, Hautfalten und/oder Hautfältchen, die Anzeichen der intrinsischen und extrinsischen Hautalterung, auf müde und/oder schlaffe Haut, trockene Haut, Altersflecken und gutartige Pigmentstörungen, fettige und/oder unreine Haut, UV-geschädigte und/oder gereizte Haut, aufgetragen wird. Ein weiterer Gegenstand der vorliegenden Erfindung ist ein Verfahren zur nicht-therapeutischen kosmetischen Hautbehandlung, bei der eine kosmetische Zusammensetzung in Form einer Creme oder Lotion auf Basis einer Wasser-in-Siliconöl-Emulsion, enthaltend enthaltend 15 - 35 Gew.-% einer Fettphase mit einem Anteil an mindestens einem Siliconöl von 70 - 95 Gew.-%, bezogen auf das Gewicht der Fettphase, Cetyl PEG/PPG-10/1 Dimethicone als Wasser-in-Öl-Siliconemulgator, 50 - 90 Gew.-% Wasserphase, bezogen auf die Gesamtzusammensetzung, 0,5 bis weniger als 3 Gew.-% mindestens einer Lipid- oder Wachskomponente, wobei der Schmelzpunkt der Lipid- oder Wachskomponente oberhalb bei 60°C oder darüber liegt oder, falls mehrere Lipid- oder Wachskomponenten enthalten sind, der Schmelzpunkt dieser Mischung von Lipid- oder Wachskomponenten bei 60°C oder darüber liegt, und wobei die Lipid- oder Wachskomponente mit einem Schmelzpunkt von 60°C oder darüber ausgewählt ist aus Estern aus einem gesättigten, einwertigen C₁₆-C₆₀-Alkanol und einer gesättigten C₈-C₃₆-Monocarbonsäure, Glycerintriestern von gesättigten linearen C₁₈ - C₄₀-Carbonsäuren, die hydroxyliert sein können, Fruchtwachsen, Pflanzenwachsen, insbesondere Candelillawachs und Carnaubawachs, Insektenwachsen, insbesondere Bienenwachs, gesättigten linearen C₁₆ -, C₁₈ -, C₂₀ - und C₂₂ - C₄₀-Carbonsäuren, gehärtetem Ricinusöl, Ozokerit, Mikrowachsen, Ceresin, Paraffinwachsen, sowie solchen Mischungen der vorgenannten Substanzen, deren Schmelzpunkt bei 60°C oder darüber liegt, und 0,5-7 Gew.-% mindestens eines Pigments mit einer mittleren Partikelgröße von 0,5 - 25 µm, das im Wellenlängenbereich von 400 - 800 nm diffuse Lichtstreuungseigenschaften und einen Transmissionswert von 60 bis mindestens 95 % aufweist und das ausgewählt ist aus sphärischen Kieselsäurepartikeln, die mit Titandioxid (CI 77891) und Eisenoxid (CI 77491) beschichtet sind, sphärischen Partikeln aus vernetztem Polymethylmethacrylat, sphärischen TiO₂-haltige Kieselsäurepartikeln sowie Mischungen der genannten Pigmente, sowie weiterhin mindestens eine anorganische oder, bevorzugt, mindestens organische UV-Filtersubstanz, auf die Haut, insbesondere die Gesichtshaut und bevorzugt auf die feinen Linien, Hautfalten und/oder Hautfältchen, die Anzeichen der intrinsischen und extrinsischen Hautalterung, auf müde und/oder schlaffe Haut, trockene Haut, Altersflecken und gutartige Pigmentstörungen, fettige und/oder unreine Haut, UV-geschädigte und/oder gereizte Haut, aufgetragen wird.
Ein weiterer Gegenstand der vorliegenden Erfindung ist ein Verfahren zur nicht-therapeutischen kosmetischen Hautbehandlung, bei der eine kosmetische Zusammensetzung in Form einer Creme oder Lotion auf Basis einer Wasser-in-Siliconöl-Emulsion, enthaltend enthaltend 15-35 Gew.-% einer Fettphase mit einem Anteil an mindestens einem Siliconöl von 70 - 95 Gew.-%, bezogen auf das Gewicht der Fettphase, Cetyl PEG/PPG-10/1 Dimethicone als Wasser-in-Öl-Siliconemulgator, 50 - 90 Gew.-% Wasserphase, bezogen auf die Gesamtzusammensetzung, 0,5 bis weniger als 3 Gew.-% mindestens einer Lipid- oder Wachskomponente, wobei der Schmelzpunkt der Lipid- oder Wachskomponente oberhalb bei 60°C oder darüber liegt oder, falls mehrere Lipid- oder Wachskomponenten enthalten sind, der Schmelzpunkt dieser Mischung von Lipid- oder Wachskomponenten bei 60°C oder darüber liegt, und wobei die Lipid- oder Wachskomponente mit einem Schmelzpunkt von 60°C oder darüber ausgewählt ist aus Estern aus einem gesättigten, einwertigen C₁₆-C₆₀-Alkanol und einer gesättigten C₈-C₃₆-Monocarbonsäure, Glycerintriestern von gesättigten linearen C₁₈ - C₄₀-Carbonsäuren, die hydroxyliert sein können, Fruchtwachsen, Pflanzenwachsen, insbesondere Candelillawachs und Carnaubawachs, Insektenwachsen, insbesondere Bienenwachs, gesättigten linearen C₁₆ -, C₁₈ -, C₂₀ - und C₂₂ - C₄₀-Carbonsäuren, gehärtetem Ricinusöl, Ozokerit, Mikrowachsen, Ceresin, Paraffinwachsen, sowie solchen Mischungen der vorgenannten Substanzen, deren Schmelzpunkt bei 60°C oder darüber liegt, und 0,5 - 7 Gew.-% mindestens eines Pigments mit einer mittleren Partikelgröße von 0,5 - 25 µm, das im Wellenlängenbereich von 400 - 800 nm diffuse Lichtstreuungseigenschaften und einen Transmissionswert von 60 bis mindestens 95 % aufweist und das ausgewählt ist aus sphärischen Kieselsäurepartikeln, die mit Titandioxid (CI 77891) und Eisenoxid (CI 77491) beschichtet sind, sphärischen Partikeln aus vernetztem Polymethylmethacrylat, sphärischen TiO₂-haltigen Kieselsäurepartikeln sowie Mischungen der genannten Pigmente, sowie weiterhin mindestens ein hautaufhellender Wirkstoff, bevorzugt 9-Z-Octadecendicarbonsäure, auf die Haut, insbesondere die Gesichtshaut und bevorzugt auf die feinen Linien, Hautfalten und/oder Hautfältchen, die Anzeichen der intrinsischen und extrinsischen Hautalterung, auf müde und/oder schlaffe Haut, trockene Haut, Altersflecken und gutartige Pigmentstörungen, fettige und/oder unreine Haut, UV-geschädigte und/oder gereizte Haut, aufgetragen wird.
Ein weiterer Gegenstand der vorliegenden Erfindung ist ein Verfahren zur nicht-therapeutischen kosmetischen Hautbehandlung, bei der eine kosmetische Zusammensetzung in Form einer Creme oder Lotion auf Basis einer Wasser-in-Siliconöl-Emulsion, enthaltend enthaltend 15-35 Gew.-% einer Fettphase mit einem Anteil an mindestens einem Siliconöl von 70 - 95 Gew.%, bezogen auf das Gewicht der Fettphase, Cetyl PEG/PPG-10/1 Dimethicone als Wasser-in-Öl-Siliconemulgator, 50 - 90 Gew.-% Wasserphase, bezogen auf die Gesamtzusammensetzung, 0,5 bis weniger als 3 Gew.-% mindestens einer Lipid- oder Wachskomponente, wobei der Schmelzpunkt der Lipid- oder Wachskomponente oberhalb bei 60°C oder darüber liegt oder, falls mehrere Lipid- oder Wachskomponenten enthalten sind, der Schmelzpunkt dieser Mischung von Lipid- oder Wachskomponenten bei 60°C oder darüber liegt, und wobei die Lipid- oder Wachskomponente mit einem Schmelzpunkt von 60°C oder darüber ausgewählt ist aus Estern aus einem gesättigten, einwertigen C₁₆-C₆₀-Alkanol und einer gesättigten C₈-C₃₆-Monocarbonsäure, Glycerintriestern von gesättigten linearen C₁₈ - C₄₀-Carbonsäuren, die hydroxyliert sein können, Fruchtwachsen, Pflanzenwachsen, insbesondere Candelillawachs und Carnaubawachs, Insektenwachsen, insbesondere Bienenwachs, gesättigten linearen C₁₆ -, C₁₈ -, C₂₀ - und C₂₂ - C₄₀-Carbonsäuren, gehärtetem Ricinusöl, Ozokerit, Mikrowachsen, Ceresin, Paraffinwachsen, sowie solchen Mischungen der vorgenannten Substanzen, deren Schmelzpunkt bei 60°C oder darüber liegt, und 0,5 - 7 Gew.-% mindestens eines Pigments mit einer mittleren Partikelgröße von 0,5 - 25 µm, das im Wellenlängenbereich von 400 - 800 nm diffuse Lichtstreuungseigenschaften und einen Transmissionswert von 60 bis mindestens 95 % aufweist und das ausgewählt ist aus sphärischen Kieselsäurepartikeln, die mit Titandioxid (CI 77891) und Eisenoxid (CI 77491) beschichtet sind, sphärischen Partikeln aus vernetztem Polymethylmethacrylat, sphärischen TiO₂-haltigen Kieselsäurepartikeln sowie Mischungen der genannten Pigmente, sowie weiterhin mindestens ein Desoxyzucker oder mindestens ein Desoxyzucker-Bausteine enthaltendes Polysaccharid, auf die Haut, insbesondere die Gesichtshaut und bevorzugt auf die feinen Linien, Hautfalten und/oder Hautfältchen, die Anzeichen der intrinsischen und extrinsischen Hautalterung, auf müde und/ oder schlaffe Haut, trockene Haut, Altersflecken und gutartige Pigmentstörungen, fettige und/oder unreine Haut, UV-geschädigte und/oder gereizte Haut, aufgetragen wird.

Weitere optionale Zusatzstoffe für die erfindungsgemäßen Zusammensetzungen sind Verdickungsmittel, z. B. natürliche und synthetische Tone und Schichtsilikate wie Bentonit, Hectorit, Montmorillonit oder Laponite^{®}, oder anionische Polymere aus Acrylsäure, Methacrylsäure, Crotonsäure, Maleinsäureanhydrid und 2-Acrylamido-2-methylpropansulfonsäure, wobei die sauren Gruppen ganz oder teilweise als Natrium-, Kalium-, Ammonium-, Mono- oder Triethanolammonium-Salz vorliegen können und wobei mindestens ein nichtionisches Monomer enthalten sein kann. Bevorzugte nichtionogene Monomere sind Acrylamid, Methacrylamid, Acrylsäureester, Methacrylsäureester, Vinylpyrrolidon, Vinylether und Vinylester. Bevorzugte anionische Copolymere sind Acrylsäure-Acrylamid-Copolymere sowie insbesondere Polyacrylamidcopolymere mit Sulfonsäuregruppen-haltigen Monomeren. Diese Copolymere können auch vernetzt vorliegen. Geeignete Handelsprodukte sind Sepigel^{®}305, Simulgel^{®}600, Simulgel^{®} NS, Simulgel^{®} EPG und Simulgel^{®} EG der Firma SEPPIC. Weitere besonders bevorzugte anionische Homo- und Copolymere sind unvernetzte und vernetzte Polyacrylsäuren. Solche Verbindungen sind zum Beispiel die Handelsprodukte Carbopol^{®}. Ein besonders bevorzugtes anionisches Copolymer enthält als Monomer zu 80 - 98 % eine ungesättigte, gewünschtenfalls substituierte C₃₋₆-Carbonsäure oder ihr Anhydrid sowie zu 2 - 20 % gewünschtenfalls substituierte Acrylsäureester von gesättigten C₁₀₋₃₀-Carbonsäuren, wobei das Copolymer mit den vorgenannten Vernetzungsagentien vernetzt sein kann. Entsprechende Handelsprodukte sind Pemulen® und die Carbopol®⁻Typen 954, 980, 1342 und ETD 2020 (ex B.F. Goodrich).
Ein weiteres besonders bevorzugtes anionisches Copolymer, das besonders günstige Verdickungseigenschaften für die erfindungsgemäßen Zusammensetzungen aufweist, ist ausgewählt aus teilweise neutralisierten 2-Acrylamido-2-methylpropansulfonsäureNinyl-pyrrolidon-Copolymeren, insbesondere einem Ammonium-2-Acrylamido-2-methylpropan-sulfonat/Vinylpyrrolidon-Copolymer, das z. B. unter der Bezeichnung Aristoflex® AVC von der Firma Clariant erhältlich ist.
Geeignete nichtionische Polymere sind beispielsweise Polyvinylalkohole, die teilverseift sein können, z. B. die Handelsprodukte Mowiol® sowie VinylpyrrolidonNinylester-Copolymere und Polyvinylpyrrolidone, die z. B. unter dem Warenzeichen Luviskol® (BASF) vertrieben werden.
Weitere geeignete Zusatzstoffe sind Antioxidantien, Konservierungsmittel, Lösungsmittel wie Ethanol, Isopropanol, Ethylenglykol, Propylenglykol, Propylenglykolmonoethylether, Glycerin und Diethylenglykol, Adsorbentien und Füllstoffe, wie Talkum und Veegum® , Parfümöle, Pigmente sowie Farbstoffe zum Anfärben des Mittels, Substanzen zur Einstellung des pH-Wertes, Komplexbildner wie EDTA, NTA, β-Alanindiessigsäure und Phosphonsäuren, Perlglanzmittel wie Ethylenglykolmono- und -distearat, Trübungsmittel und Treibmittel wie Propan-Butan-Gemische, N₂O, Dimethylether, CO₂ und Luft.
In einer besonders bevorzugten Ausführungsform können die erfindungsgemäßen Zusammensetzungen mit einem Treibgas abgefüllt und als Mousse oder Aerosolcreme konfektioniert sein.
Die nachfolgenden Beispiele sollen den Gegenstand der Erfindung erläutern, ohne ihn hierauf zu beschränken.

### Wirkungsnachweis

Zum Nachweis, dass der hautfaltenkaschierende Effekt von Pigmenten mit einer mittleren Partikelgröße von 0,5 - 25 µm, die im Wellenlängenbereich von 400 - 800 nm diffuse Lichtstreuungseigenschaften und einen Transmissionswert von 60 bis mindestens 95 % aufweisen, durch die Einarbeitung in eine erfindungsgemäße Creme oder Lotion auf Basis einer Wasser-in-Siliconöl-Emulsion verbessert wird im Vergleich zu einer Öl-in-Wasser-Emulsion, wurden die erfindungsgemäß bevorzugten kaschierenden Pigmente zum einen in eine erfindungsgemäße W/O-Emulsion und zum Vergleich in eine Öl-in-Wasser-Emulsion eingearbeitet.

Zur Bewertung der optischen Hautglättung wurden die zu testenden Emulsionen von 6 Probanden einmalig am Unterarm angewendet und das Oberflächenprofil mittels Visioscan (Courage & Khazaka) analysiert. Bei diesem Verfahren werden mittels einer CCD-Kamera Bilder der Hautoberfläche aufgenommen und über einen Bildspeicher in verschiedene Graustufen umgewandelt. Über die Analyse dieser Graustufenverteilung können Höhen und Tiefen in einer Aufnahme und somit das Profil indirekt berechnet werden. Die SELS-Software (SELS = surface evaluation of living skin) berechnet automatisch verschiedene Parameter wie Schuppigkeit, Rauigkeit, Faltigkeit und Glätte. Für die erfindungsgemäße Fragestellung wurde der Parameter S_{EW} (Faltigkeit/Wrinkles) herangezogen. S_{EW} errechnet sich aus Anzahl, Breite und Verhältnis von horizontalen und vertikalen Falten.

Folgende Rezepturen wurden hergestellt:
1. erfindungsgemäße Wasser-in-Siliconöl-Emulsionen (Mengen in Gew.-%)

| | W/Si Nr. 1 | W/Si Nr. 2 | W/Si Nr. 3 |
|---|---|---|---|
| Belsil DM 100 | 2,50 | 2,50 | 2,50 |
| DC 245 Fluid | 25,00 | 25,00 | 25,00 |
| Abil EM 90 | 2,00 | 2,00 | 2,00 |
| Cutina HR | 2,00 | 2,00 | 2,00 |
| NaCl | 2,00 | 2,00 | 2,00 |
| Perfume | 0,30 | 0,30 | 0,30 |
| Chlorhexidindigluconat | 0,20 | 0,20 | 0,20 |
| Ronasphere LDP | 2,00 | - | - |
| SUNPMMA-S | - | 2,00 | - |
| SunSil Tin 30 | - | - | 2,00 |
| Glycerin, 86%ig | 5,00 | 5,00 | 5,00 |
| Wasser | ad 100 | ad 100 | ad 100 |

2. Vergleichsbeispiele (O/W-Rezepturen) (Mengen in Gew.-%)

| | O/W + 2% Ronasphere LDP | O/W + 2% SunSil Tin 30 |
|---|---|---|
| Lipoid S75-3 | 0,50 | 0,50 |
| Isopropylstearat | 4,00 | 4,00 |
| Cetiol B | 2,00 | 2,00 |
| Tocopherylacetat | 0,50 | 0,50 |
| Cutina MD | 1,00 | 1,00 |
| Stenol 1618 | 0,50 | 0,50 |
| Lanette 22 | 2,00 | 2,00 |
| Baysilon M 350 | 0,50 | 0,50 |
| Propylparaben | 0,20 | 0,20 |
| Dow Corning 9040 | 1,00 | 1,00 |
| Glycerin, 86 %ig | 3,00 | 3,00 |
| Hexandiol | 3,00 | 3,00 |
| Methylparaben | 0,20 | 0,20 |
| Tego Carbomer 140 | 0,30 | 0,30 |
| DSH-CN | 5,00 | 5,00 |
| Algenextrakt | 1,00 | 1,00 |
| Photosomes | 0,20 | 0,20 |
| Lipochroman-6 | 0,01 | 0,01 |
| Propylenglycol | 5,00 | 5,00 |
| Matrixyl 3000 | 3,00 | 3,00 |
| Phenoxyethanol | 0,40 | 0,40 |
| Simulgel NS | 1,50 | 1,50 |
| Ronasphere LDP | 2,00 | - |
| SunSil Tin 30 | - | 2,00 |
| Perfum | 0,20 | 0,20 |
| Aqua | ad 100 | ad 100 |

### Ergebnis

Die Applikation aller Rezepturen führte zu einer Abnahme des Parameters S_{EW} (Tabelle 3). Die Ausgangslagendifferenzen der erfindungsgemäßen W/Si-Formulierungen stellte sich besser dar als die Ausgangslagendifferenzen der jeweils vergleichbaren O/W-Systeme.

**Tabelle 3**

| | S_{EW} (t₀-t₅ₘᵢₙ) | Ranking bezogen auf Kontrolle |
|---|---|---|
| W/Si ohne Pigmente (Kontrolle) | -3,15 | - |
| W/Si +2% Ronasphere LDP | -4,24 | 1 |
| O/W + 2% Ronasphere LDP | -2,28 | 4 |
| W/Si +2% SunSil Tin 30 | -2,37 | 3 |
| O/W + 2% SunSil Tin 30 | -1,79 | 5 |
| W/Si +2% SUNPMMA-S | -3,69 | 2 |

Ergänzend zur biophysikalischen Messung mittels Visioscan wurden die Prüfmuster bei 3 Probanden mit Fältchen im Augenbereich einmalig appliziert und Bildaufnahmen mit einer CCD-Kamera vor sowie 5 Minuten nach der Applikation erstellt. Im Anschluss wurden die Fotos von Experten begutachtet und gewichtet (Tabelle 4). Auch mit dieser Methode zeigt sich gegenüber dem O/W-System eine deutliche Verbesserung der optischen Hautglättung, wenn die Pigmente in eine erfindungsgemäße W/Si-Emulsion eingearbeitet sind.

**Tabelle 4: Ergebnisse der Fotoauswertung**

| | Häufigkeit und Ausprägungsgrad der optischen Glättung | Ranking |
|---|---|---|
| W/Si ohne Pigmente (Kontrolle) | 1 von 3 Probanden mit leichten Effekten | 6 |
| W/Si +2% Ronasphere LDP | 3 von 3 Probanden mit deutlichen Effekten | 3 |
| O/W + 2% Ronasphere LDP | 2 von 3 Probanden mit Effekten | 5 |
| W/Si +2% SunSil Tin 30 | 3 von 3 Probanden mit deutlichen Effekten | 2 |
| O/W + 2% SunSil Tin 30 | 2 von 3 Probanden mit z.T. deutlichen Effekten | 4 |
| W/Si +2% SUNPMMA-S | 3 von 3 Probanden mit sehr deutlichen Effekten | 1 |

### Weitere Formulierungsbeispiele: Tagespflege ohne UV-Schutz

| | 1 | 2 | 3 | 4 | 5 |
|---|---|---|---|---|---|
| Belsil DM 100 | 2,50 | 2,50 | 2,50 | 2,50 | 5,00 |
| DC 245 Fluid | 25,00 | 25,00 | 25,00 | - | - |
| DC 2-1145 Fluid | - | - | - | 25,00 | 20,00 |
| Abil EM 90 | 2,00 | 2,00 | 2,00 | 2,00 | 2,00 |
| Cutina HR | 2,00 | 1,50 | 1,00 | 2,00 | 1,50 |
| Natriumchlorid | 2,00 | 2,00 | 2,00 | 2,00 | 2,00 |
| Parfüm | 0,30 | 0,30 | 0,30 | 0,30 | 0,30 |
| Methylparaben | 0,20 | 0,20 | 0,20 | - | - |
| Propylparaben | 0,20 | 0,20 | 0,20 | - | - |
| Phenoxyethanol | 0,40 | 0,40 | 0,40 | - | - |
| Chlorhexidindigluconat | - | - | - | 0,20 | 0,20 |
| Glycerin, 86 %ig | 5,00 | 2,50 | 5,00 | 5,00 | 2,50 |
| (2-Hydroxyethyl)harnstoff | - | 8,60 | 2,50 | 4,30 | 8,60 |
| Taurin | - | 1,00 | - | 1,00 | - |
| Matrixyl 3000 | 3,00 | | 3,00 | | |
| Phytokine | | 2,00 | | | |
| Ederline L | | | 2,00 | 2,00 | |
| Ultrasomes | | | 0,10 | | |
| Photosomes | | | | 0,10 | |
| SUNPMMA -S | 2,00 | - | - | 2,00 | - |
| SunSil Tin 30 | - | 2,00 | - | - | 2,00 |
| Ronasphere LDP | - | - | 2,00 | - | - |
| Wasser | ad 100 | ad 100 | ad 100 | ad 100 | ad 100 |

Die Zusammensetzungen 1 - 5 wurden auf die Gesichtshaut aufgetragen. Einige Probandinnen wendeten die Zusammensetzung einmal täglich, andere zweimal täglich (morgens und abends) an.

Direkt nach der Anwendung ließ sich eine optische Kaschierung von feinen Linien, Hautfalten und/oder Hautfältchen, Altersflecken und gutartigen Pigmentstörungen erkennen.
Darüber hinaus konnte nach regelmäßiger Anwendung über vier Wochen festgestellt werden, dass sich das Erscheinungsbild der vor Behandlungsbeginn müden und/oder schlaffen Haut bzw. der vor Behandlungsbeginn trockenen Haut deutlich verbessert hatte.
Bei einigen Probandinnen konnte bei regelmäßiger Anwendung nach vier Wochen eine Verbesserung des Erscheinungsbildes der vor Behandlungsbeginn fettigen und/oder unreinen Haut festgestellt werden.
Bei einigen Probandinnen konnte bei regelmäßiger Anwendung nach vier Wochen eine Verbesserung des Erscheinungsbildes der vor Behandlungsbeginn UV-geschädigten und/oder gereizten Haut festgestellt werden.

### Weitere Formulierungsbeispiele: Tagespflege mit UV-Schutz

| | 6 | 8 | 13 |
|---|---|---|---|
| Belsil DM 100 | 2,50 | 2,50 | 2,50 |
| DC 245 Fluid | 25,00 | 25,00 | 25,00 |
| Abil EM 90 | 2,00 | 2,00 | 2,00 |
| Cutina HR | 2,00 | 2,00 | - |
| Kester Wachs K 80 H | - | - | 2,00 |
| Parsol SLX | 4,00 | - | - |
| Phenylbenzimidazole Sulfonic Acid | - | 2,00 | - |
| Disodium Phenyl Dibenzimidazole Tetrasulfonate | - | 1,00 | - |
| Octocrylene | - | 5,00 | - |
| Octyl Triazone | - | - | - |
| Octylsalicylate | - | - | - |
| Diethylamino Hydroxy-benzoyl Hexyl Benzoat | - | - | - |
| 4-Methoxy benzylidene Camphor | - | - | 2,00 |
| Butyl Methoxy-dibenzoylmethane | - | - | 1,00 |
| Sodium Chloride | 2,00 | 2,00 | 2,00 |
| Perfume | 0,30 | 0,30 | 0,30 |
| Methylparaben | 0,20 | 0,20 | - |
| Propylparaben | 0,20 | 0,20 | - |
| Phenoxyethanol | 0,40 | 0,40 | - |
| Chlorhexidinedigluconat | - | - | 0,20 |
| Glycerin, 86 %ig | 5,00 | 5,00 | 5,00 |
| (2-Hydroxyethyl)urea | - | 2,50 | - |
| Taurin | - | - | - |
| Matrixyl 3000 | 3,00 | 3,00 | |
| Phytokine | | | |
| Ederline L | | 2,00 | |
| Ultrasomes | | | 0,10 |
| Photosomes | | 0,10 | |
| SUNPMMA-S | 2,00 | - | |
| SunSil Tin 30 | - | - | - |
| Ronasphere LDP | - | 2,00 | 2,00 |
| Aqua | ad 100 | ad 100 | ad 100 |

Die Zusammensetzungen 6, 8 und 13 wurden auf die Gesichtshaut aufgetragen. Einige Probandinnen wendeten die Zusammensetzung einmal täglich, andere zweimal täglich (morgens und abends) an.
Direkt nach der Anwendung ließ sich eine optische Kaschierung von feinen Linien, Hautfalten und/oder Hautfältchen, Altersflecken und gutartigen Pigmentstörungen erkennen. Darüber hinaus konnte nach regelmäßiger Anwendung über vier Wochen festgestellt werden, dass sich das Erscheinungsbild der vor Behandlungsbeginn müden und/oder schlaffen Haut bzw. der vor Behandlungsbeginn trockenen Haut deutlich verbessert hatte.
Bei einigen Probandinnen konnte bei regelmäßiger Anwendung nach vier Wochen eine Verbesserung des Erscheinungsbildes der vor Behandlungsbeginn UV-geschädigten und/oder gereizten Haut festgestellt werden.

### Verwendete Rohstoffe

| Abil EM 90 | Cetyl Dimethicone Copolyol | Wacker |
|---|---|---|
| Baysilonöl M 350 | Dimethicone | GE Bayer Silicones |
| Belsil DM 100 | Dimethicone | Wacker |
| Cetiol B | Diisopropyladipat | Cognis |
| Cutina HR | Hydrogenated Castor Oil | Cognis |
| Cutina MD | Glycerylmono-, -distearat | Cognis |
| DC 245 Fluid | Cyclomethicone | Dow Corning |
| DC 2-1145 Fluid | Trisiloxane and Dimethicone | Dow Corning |
| Dow Corning 9040 | Cyclomethicone, Dimethicone Crosspolymer | Dow Corning |
| DSH-CN | Dimethylsilanol Hyaluronate | Exsymol |
| Ederline L | Hexyldecanol, Pyrus Malus (Apple) Fruit Extract | Vincience |
| (2-Hydroxyethyl)harnstoff | (2-Hydroxyethyl)urea = enthalten im Rohstoff Hydrovance, enthält N,N'-Bis(2-Hydroxyethyl)harnstoff | National Starch |
| Kester Wachs K 80 H | Synthetic Beeswax | Koster Keunen |
| Lanette 22 | Behenyl alcohol | Cognis |
| Lipochroman-6 | Dimethylmethoxy Chromanol | Lipotec |
| Lipoid S75-3 | Hydrogenated Lecithin | Lipoid GmbH |
| Matrixyl 3000 | Glycerin, Aqua (Water), Butylene Glycol, Carbomer, Polysorbate-20, Palmitoyl Oligopeptide, Palmitoyl Tetrapeptide-3 | Sederma |
| Parsol SLX | Dimethicodiethylbenzalmalonate, Methyl Alcohol, Alcohol | Roche Vitamins |
| Photosomes | Aqua (Water), Lecithin, Plankton Extract | Barnet |
| Ronasphere LDP | Silicium dioxide, Titanium dioxide, Iron oxide | Merck KGaA |
| Phytokine | Hydrolyzed Soy Protein | Coletica |
| Simulgel NS | Hydroxyethyl Acrylate/Sodium Acryloyldimethyl Taurate Copolymer, Squalane, Polysorbate-60 | Seppic |
| Stenol 1618 | Cetearyl alcohol | Cognis |
| SUNPMMA-S | Methyl Methacrylate Cross Polymer | Sunjin Chemical Co. |
| SunSil Tin 30 | Silicium dioxide, Titanium dioxide | Sunjin Chemical Co. |
| Tego Carbomer 140 | Carbomer | Goldschmidt |
| Ultrasomes | Aqua (Water), Lecithin, Micrococcus Lysate | Barnet |

## Patentansprüche

1. Kosmetische Zusammensetzung in Form einer Creme oder Lotion auf Basis einer Wasser-in-Siliconöl-Emulsion, enthaltend:
- 15-35 Gew.-% einer Fettphase mit einem Anteil an mindestens einem Siliconöl von 70 - 95 Gew.-%, bezogen auf das Gewicht der Fettphase,
- Cetyl PEG/PPG-10/1 Dimethicone als Wasser-in-Öl-Siliconemulgator,
- 50 - 90 Gew.-% Wasserphase, bezogen auf die Gesamtzusammensetzung,
- 0,5 bis weniger als 3 Gew.-% mindestens einer Lipid- oder Wachskomponente, wobei der Schmelzpunkt der Lipid- oder Wachskomponente oberhalb bei 60°C oder darüber liegt oder, falls mehrere Lipid- oder Wachskomponenten enthalten sind, der Schmelzpunkt dieser Mischung von Lipid- oder Wachskomponenten bei 60°C oder darüber liegt, und wobei die Lipid- oder Wachskomponente mit einem Schmelzpunkt von 60°C oder darüber ausgewählt ist aus Estern aus einem gesättigten, einwertigen C₁₆-C₆₀-Alkanol und einer gesättigten C₈-C₃₆-Monocarbonsäure, Glycerintriestern von gesättigten linearen C₁₈ - C₄₀-Carbonsäuren, die hydroxyliert sein können, Fruchtwachsen, Pflanzenwachsen, insbesondere Candelillawachs und Carnaubawachs, Insektenwachsen, insbesondere Bienenwachs, gesättigten linearen C₁₆ -, C₁₈ -, C₂₀ - und C₂₂ - C₄₀-Carbonsäuren, gehärtetem Ricinusöl, Ozokerit, Mikrowachsen, Ceresin, Paraffinwachsen, sowie solchen Mischungen der vorgenannten Substanzen, deren Schmelzpunkt bei 60°C oder darüber liegt,
- 0,5 - 7 Gew.-% mindestens eines Pigments mit einer mittleren Partikelgröße von 0,5 - 25 µm, das im Wellenlängenbereich von 400 - 800 nm diffuse Lichtstreuungseigenschaften und einen Transmissionswert von 60 bis mindestens 95 % aufweist und das ausgewählt ist aus sphärischen Kieselsäurepartikeln, die mit Titandioxid (CI 77891) und Eisenoxid (CI 77491) beschichtet sind, sphärischen Partikeln aus vernetztem Polymethylmethacrylat, sphärischen TiO₂-haltige Kieselsäurepartikeln sowie Mischungen der genannten Pigmente.

2. Kosmetische Zusammensetzung gemäß Anspruch 1, **dadurch gekennzeichnet, dass** die mindestens eine Lipid- oder Wachskomponente mit einem Schmelzpunkt von 60°C oder darüber in einer Menge von 1,0 - 2,5 Gew.%, bezogen auf die Gesamtzusammensetzung, enthalten ist.

3. Kosmetische Zusammensetzung gemäß Anspruch 1 oder 2, **dadurch gekennzeichnet, dass** der Schmelzpunkt der mindestens einen Lipid- oder Wachskomponente oder der Mischung der Lipid- oder Wachskomponenten einen Schmelzpunkt von 70 °C oder darüber, bevorzugt von 78 °C oder darüber, aufweist.

4. Kosmetische Zusammensetzung gemäß einem der Ansprüche 1 - 3, **dadurch gekennzeichnet, dass** die mindestens eine Lipid- oder Wachskomponente mit einem Schmelzpunkt von 60°C oder darüber gehärtetes Ricinusöl ist.

5. Kosmetische Zusammensetzung gemäß einem der Ansprüche 1-4, **dadurch gekennzeichnet, dass** die Fettphase 20 - 32 Gew.-% an der Gesamtzusammensetzung ausmacht.

6. Kosmetische Zusammensetzung gemäß einem der Ansprüche 1-5, **dadurch gekennzeichnet, dass** die Fettphase einen Anteil von 80 - 95 Gew.-%, bezogen auf das Gewicht der Fettphase, an mindestens einem Siliconöl aufweist.

7. Kosmetische Zusammensetzung gemäß einem der Ansprüche 1-6, **dadurch gekennzeichnet, dass** das mindestens eine Pigment in einer Menge von 1 - 4 Gew.-% und bevorzugt 2-3 Gew.%, jeweils bezogen auf die Gesamtzusammensetzung, enthalten ist.

8. Kosmetische Zusammensetzung gemäß einem, der Ansprüche 1 - 7, **dadurch gekennzeichnet, dass** mindestens ein weiterer kosmetischer Wirkstoff enthalten ist, ausgewählt aus:
a) Feuchthaltemitteln,
b) Monomeren, Oligomeren und Polymeren von Aminosäuren, N-C₂-C₂₄-Acylaminosäuren, den Estern und/oder den physiologisch verträglichen Metallsalzen dieser Substanzen,
c) DNA- oder RNA-Oligonucleotiden,
d) natürlichen Betainverbindungen,
e) Vitaminen, Provitaminen und Vitaminvorstufen der Gruppen A, B, C, E, H und K und den Estern der vorgenannten Substanzen,
f) α-Hydroxycarbonsäuren, α-Ketocarbonsäuren, β-Hydroxycarbonsäuren und deren Ester-, Lacton- oder Salzform,
g) Flavonoiden und Flavonoid-reichen Pflanzenextrakten,
h) Isoflavonoiden und Isoflavonoid-reichen Pflanzenextrakten,
i) Polyphenolen und Polyphenol-reichen Pflanzenextrakten,
j) Ubichinon und Ubichinol sowie deren Derivaten,
k) Silymarin,
l) natürlich vorkommenden Xanthin-Derivaten, ausgewählt aus Coffein, Theophyllin, Theobromin und Aminophyllin,
m) Ectoin,
n) anorganischen und organischen UV-Filtersubstanzen,
o) selbstbräunenden Wirkstoffen,
p) hautaufhellenden Wirkstoffen,
q) hautberuhigenden Wirkstoffen,
r) sowie Desoxyzuckern oder Desoxyzucker-Bausteine enthaltenden Polysäcchariden.

9. Kosmetische Zusammensetzung gemäß einem der Ansprüche 1-8, **dadurch gekennzeichnet, dass** sie mit einem Treibgas abgefüllt und als Mousse oder Aerosolcreme konfektioniert ist.

10. Verwendung einer kosmetischen Zusammensetzung gemäß einem der vorhergehenden Ansprüche zur nicht-therapeutischen, kosmetischen Behandlung und/oder optischen Kaschierung von
- feinen Linien, Hautfalten und/oder Hautfältchen,
- den Anzeichen der intrinsischen und extrinsischen Hautalterung,
- müder und/oder schlaffer Haut,
- trockener Haut,
- Altersflecken und gutartigen Pigmentstörungen,
- fettiger und/oder unreiner Haut,
- UV-geschädigter Haut,
- gereizter Haut.

11. Verfahren zur nicht-therapeutischen kosmetischen Hautbehandlung, **dadurch gekennzeichnet, dass** eine kosmetische Zusammensetzung gemäß einem der Ansprüche 1 - 9 auf die Haut, insbesondere die Gesichtshaut und bevorzugt auf die feinen Linien, Hautfalten und/oder Hautfältchen, die Anzeichen der intrinsischen und extrinsischen Hautalterung, auf müde und/oder schlaffe Haut, trockene Haut, Altersflecken und gutartige Pigmentstörungen, fettige und/oder unreine Haut, UV-geschädigte und/oder gereizte Haut, aufgetragen wird.

## Claims

1. A cosmetic composition in the form of a cream or lotion based on a water-in-silicone-oil emulsion, containing:
- 15 to 35% by weight of a fatty phase with a proportion of at least one silicone oil from 70 to 95% by weight, based on the weight of the fatty phase,
- cetyl PEG/PPG-10/1 dimethicone as a water-in-oil silicone emulsifier,
- 50 to 90% by weight of an aqueous phase, based on the total composition,
- 0.5 to less than 3% by weight of at least one lipid or wax component, the melting point of the lipid or wax component lying over 60°C or above, in the case of several lipid or wax components being contained, the melting point of this mixture of lipid or wax components lying at 60°C or above, and the lipid or wax component with a melting point of 60°C or above is selected from esters derived from a monovalent saturated C₁₆-C₆₀ alkanol and a saturated C₈-C₃₆ monocarboxylic acid, from glycerol triesters of saturated linear C₁₈-C₄₀ carboxylic acids, which may be hydroxylated, from fruit waxes, plant waxes, in particular candelilla wax and carnauba wax, insect waxes, in particular beeswax, saturated linear C₁₆-C₁₈, C₂₀ and C₂₂-C₄₀ carboxylic acids, hardened castor oil, ozokerite, microwaxes, ceresin, paraffin waxes, as well as such mixtures of the aforementioned substances, for which the melting point lies at 60°C or above,
- 0.5 to 7% by weight of at least one pigment with an average particle size from 0.5 to 25 µm, which has diffuse light scattering properties and a transmission value from 60 to at least 95% in the wavelength range from 400 to 800 nm and which is selected from spherical silicic acid particles which are coated with titanium dioxide (CI 77891) and iron oxide (CI 77491), from spherical particles of cross-linked polymethyl methacrylate, spherical TiO₂-containing silicic acid particles as well as mixtures of the mentioned pigments.

2. The cosmetic composition according to claim 1, **characterized in that** said at least one lipid or wax component with a melting point of 60°C or above is contained in an amount from 1.0 to 2.5% by weight, based on the total composition.

3. The cosmetic composition according to claim 1 or 2, **characterized in that** the melting point of said at least one lipid or wax component or of the mixture of lipid or wax components has a melting point of 70°C or above, preferably of 78°C or above.

4. The cosmetic composition according to any of claims 1 to 3, **characterized in that** said at least one lipid or wax component with a melting point of 60°C or above, is hardened castor oil.

5. The cosmetic composition according to any of claims 1 to 4, **characterized in that** the fatty phase makes up 20 to 32% by weight of the total composition.

6. The cosmetic composition according to any of claims 1 to 5, **characterized in that** the fatty phase has a proportion of 80 to 95% by weight, based on the weight of the fatty phase, of at least one silicone oil.

7. The cosmetic composition according to any of claims 1 to 6, **characterized in that** said at least one pigment is contained in an amount of 1 to 4% by weight and preferably from 2 to 3% by weight, respectively based on the total composition.

8. The cosmetic composition according to any of claims 1 to 7, **characterized in that** at least one additional cosmetic active ingredient is contained, selected from:
a) humectants
b) monomers, oligomers and polymers of aminoacids, N-(C₂-C₂₄-acyl)-aminoacids, the esters and/or the physiologically acceptable metal salts of these substances,
c) DNA- or RNA-oligonucleotides,
d) natural betaine compounds,
e) vitamins, provitamins and vitamin precursors of the A, B, C, E, H and K groups and the esters of the aforementioned substances,
f) α-hydroxycarboxylic acids, α-ketocarboxylic acids, β-hydroxycarboxylic acids and their ester, lactone or salt form,
g) flavonoids and plant extracts rich in flavonoids,
h) isoflavonoids and plant extracts rich in isoflavonoids,
i) polyphenols and plant extracts rich in polyphenols,
j) ubiquinone and ubiquinol as well as their derivatives,
k) silymarin,
l) naturally occurring xanthine derivatives, selected from caffeine, theophylline, theobromine and aminophylline,
m) ectoine,
n) inorganic and organic UV filter substances,
o) self-tanning active ingredients,
p) skin-lightening active ingredients,
q) skin-soothing active ingredients,
r) as well as deoxy sugars or polysaccharides containing deoxy sugar constituents.

9. The cosmetic composition according to any of claims 1 to 8, **characterized in that** it is filled with a propellant gas and made ready-for-use as a foam or an aerosol cream.

10. The use of a cosmetic composition according to any of the preceding claims for non-therapeutic, cosmetic treatment and/or optical concealment of
- fine lines, skin wrinkles and/or skin crow's feet,
- signs of intrinsic and extrinsic skin ageing,
- tired and/or flabby skin,
- dry skin,
- age spots and benign pigment disorders,
- fatty and/or impure skin,
- UV-damaged skin,
- irritated skin.

11. A method for non-therapeutic cosmetic skin treatment, **characterized in that** a cosmetic composition according to any of claims 1 to 9 is applied on the skin, in particular on the face skin and preferably on the fine lines, skin wrinkles and/or skin crow's feet, the signs of intrinsic and extrinsic skin ageing, on tired and/or flabby skin, dry skin, age spots and benign pigment disorders, fatty and/or impure skin, UV-damaged and/or irritated skin.

## Revendications

1. Composition cosmétique sous la forme d'une crème ou d'une lotion à base d'une émulsion du type eau-dans-huile-de-silicone, contenant :
- à concurrence de 15 à 35 % en poids, une phase grasse possédant une teneur en au moins une huile de silicone s'élevant de 70 à 95 % en poids, rapportés au poids de la phase grasse ;
- du Cetyl PEG/PPG-10/1 Dimethicone à titre d'émulsifiant de silicone du type eau-dans-huile;
- à concurrence de 50 à 90 % en poids, une phase aqueuse, rapportés à la composition totale ;
- à concurrence de 0,5 à moins de 3 % en poids, au moins un composant lipidique ou cireux, le point de fusion du composant lipidique ou cireux étant supérieur à 60 °C ou plus ou bien, dans le cas d'une présence de plusieurs composants lipidiques ou cireux, le point de fusion de ce mélange de composant lipidique ou cireux s'élevant à 60 °C ou plus, et le composant lipidique ou cireux possédant un point de fusion de 60 °C ou plus étant choisi parmi des esters d'un alcool monovalent saturé en C₁₆-C₆₀ et un acide monocarboxylique saturé en C₈-C₃₆, des triesters de glycérol d'acides carboxyliques linéaires saturés en C₁₈-C₄₀, qui peuvent être hydroxylés, des cires de fruits, des cires végétales, en particulier la cire de candellila et la cire de carnauba, des cires d'insectes, en particulier la cire d'abeille, des acides carboxyliques linéaires saturés en C₁₆-, C₁₈-, C₂₀- et C₂₂-C₄₀, de l'huile de ricin durcie, de l'ozokérite, des microcires, de la cérésine, des cires de paraffine, ainsi que des mélanges de substances susmentionnées dont le point de fusion s'élève à 60 °C ou plus ;
- à concurrence de 0,5 à 7 % en poids, au moins un pigment possédant une granulométrie moyenne de 0,5 à 25 µm, qui présente, dans la plage de longueur d'onde de 400 à 800 nm, des propriétés de diffusion de lumière diffuse et un taux de transmission de 60 jusqu'à au moins 95 % et qui est choisi parmi des particules sphériques d'acide silicique qui sont enduites de dioxyde de titane (CI 77891) et d'oxyde de fer (CI 77491), des particules sphériques de polyméthacrylate de méthyle réticulé, des particules sphériques d'acide silicique contenant du TiO₂, ainsi que des mélanges des pigments mentionnés.

2. Composition cosmétique selon la revendication 1, **caractérisée en ce que** ledit au moins un composant lipidique ou cireux possédant un point de fusion de 60 °C ou plus est contenu en une quantité de 1,0 à 2,5 % en poids, rapportés à la composition totale.

3. Composition cosmétique selon la revendication 1 ou 2, **caractérisée en ce que** le point de fusion dudit au moins un composant lipidique ou cireux ou du mélange des composants lipidiques ou cireux présente un point de fusion de 70 °C ou plus, de préférence de 78 °C ou plus.

4. Composition cosmétique selon l'une quelconque des revendications 1 à 3, **caractérisée en ce que** ledit au moins un composant lipidique ou cireux possédant un point de fusion de 60 °C ou plus est de l'huile de ricin durcie.

5. Composition cosmétique selon l'une quelconque des revendications 1 à 4, **caractérisée en ce que** la phase grasse représente de 20 à 32 % en poids de la composition totale.

6. Composition cosmétique selon l'une quelconque des revendications 1 à 5, **caractérisée en ce que** la phase grasse présente une fraction à concurrence de 80 à 95 % en poids, rapportés au poids de la phase grasse, d'au moins une huile de silicone.

7. Composition cosmétique selon l'une quelconque des revendications 1 à 6, **caractérisée en ce qu'**elle contient au moins un pigment en une quantité de 1 à 4 % en poids et de préférence de 2 à 3 % en poids, chaque fois rapportés à la composition totale.

8. Composition cosmétique selon l'une quelconque des revendications 1 à 7, **caractérisée en ce qu'**elle contient au moins une substance active cosmétique supplémentaire, choisie parmi :
a) des agents qui maintiennent l'humidité ;
b) des monomères, des oligomères et des polymères d'acides aminés, d'acides N-acyl(en C₂-C₂₄)aminés, les esters et/ou les sels métalliques physiologiquement acceptables de ces substances ;
c) des oligonucléotides d'ADN ou d'ARN ;
d) des composés de bétaïne naturelle ;
e) des vitamines, des provitamines et des précurseurs de vitamines des groupes A, B, C, E, H et K et les esters des substances susmentionnées ;
f) des acides α-hydroxycarboxyliques, des acides α-cétocarboxyliques, des acides β-hydroxycarboxyliques, ainsi que leur forme d'ester, de lactone ou de sel ;
g) des flavonoïdes et des extraits de plantes riches en flavonoïdes ;
h) des isoflavonoïdes et des extraits de plantes riches en isoflavonoïdes ;
i) des polyphénols et des extraits de plantes riches en polyphénols ;
j) de l'ubiquinone et de l'ubiquinol ainsi que leurs dérivés ;
k) de la silymarine ;
l) des dérivés de xanthine existant naturellement, choisi parmi la caféine, la théophylline, la théobromine et l'aminophylline ;
m) l'ectoïne ;
n) des substances inorganiques et organiques filtrant le rayonnement ultraviolet ;
o) des substances actives autobronzantes ;
p) des substances actives éclaircissant la peau ;
q) des substances actives apaisantes pour la peau ;
r) ainsi que des désoxysucres ou des polysaccharides contenant des constituants de désoxysucres.

9. Composition cosmétique selon l'une quelconque des revendications 1 à 8, **caractérisée en ce qu'**elle est transvasée avec un gaz de moussage et est confectionnée sous la forme d'une mousse ou d'une crème d'aérosol.

10. Utilisation d'une composition cosmétique selon l'une quelconque des revendications précédentes, pour le traitement cosmétique non thérapeutique et/ou pour le revêtement optique
- des petits sillons, des rides et/ou des ridules ;
- des manifestations d'une modification cutanée intrinsèque et extrinsèque ;
- de la peau fatiguée et/ou flasque ;
- de la peau sèche ;
- des taches de vieillesse et des lésions dyschromatiques bénignes ;
- de la peau grasse et/ou chargée d'impuretés ;
- de la peau endommagée par l'effet du rayonnement UV ;
- de la peau irritée.

11. Procédé pour le traitement cutané cosmétique non thérapeutique, **caractérisé en ce qu'**on applique une composition cosmétique selon l'une quelconque des revendications 1 à 9, sur la peau, en particulier sur la peau du visage et de préférence sur les petits sillons, les rides et/ou les ridules, sur les manifestations d'une modification cutanée intrinsèque et extrinsèque, sur la peau fatiguée et/ou flasque, sur la peau sèche, sur des taches de vieillesse et/ou des lésions dyschromatiques bénignes, sur la peau grasse et/ou chargée d'impuretés, sur la peau endommagée par l'effet du rayonnement UV et/ou sur la peau irritée.
